(19) Europäisches Patentamt
European Patent Office
Office européen des brevets

(11) **EP 3 114 443 B1**

(12) **EUROPÄISCHE PATENTSCHRIFT**

(45) Veröffentlichungstag und Bekanntmachung des Hinweises auf die Patenterteilung:
**01.05.2024   Patentblatt 2024/18**

(21) Anmeldenummer: **15717427.7**

(22) Anmeldetag: **06.03.2015**

(51) Internationale Patentklassifikation (IPC):
**G01J 3/02** (2006.01)   **G01J 3/10** (2006.01)
**G01J 3/42** (2006.01)   **A61B 5/145** (2006.01)
**A61B 5/1455** (2006.01)   **E01C 23/08** (2006.01)
**E21C 35/197** (2006.01)   **G01N 21/3554** (2014.01)
**G01N 21/31** (2006.01)   **G01N 21/33** (2006.01)
**G01N 21/49** (2006.01)

(52) Gemeinsame Patentklassifikation (CPC):
**E01C 23/08; A61B 5/14535; A61B 5/1455; E21C 35/197; G01J 3/0218; G01J 3/0262; G01J 3/0286; G01J 3/0291; G01J 3/0294; G01J 3/0297; G01J 3/42; G01N 21/3554; G01N 21/49;** G01J 2003/104; G01N 21/33;  (Forts.)

(86) Internationale Anmeldenummer:
**PCT/EP2015/054795**

(87) Internationale Veröffentlichungsnummer:
**WO 2015/132412 (11.09.2015 Gazette 2015/36)**

(54) **SENSORVORRICHTUNG FÜR ORTSAUFLÖSENDE ERFASSUNG VON ZIELSUBSTANZEN**

SENSOR DEVICE FOR HIGH-RESOLUTION DETECTION OF TARGET SUBSTANCES

DISPOSITIF DE DÉTECTION CONÇU POUR UNE DÉTECTION À RÉSOLUTION SPATIALE DE SUBSTANCES CIBLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.03.2014   DE 102014003470**

(43) Veröffentlichungstag der Anmeldung:
**11.01.2017   Patentblatt 2017/02**

(73) Patentinhaber: **Courage + Khazaka Electronic GmbH**
**50829 Köln (DE)**

(72) Erfinder:
• **HELFMANN, Jürgen**
**14532 Klein-Machnow (DE)**
• **CAPPIUS, Hans-Joachim**
**12157 Berlin (DE)**

(74) Vertreter: **Dantz, Dirk**
**Dantz IP & Law**
**Kurfürstendamm 43**
**10719 Berlin (DE)**

(56) Entgegenhaltungen:
**EP-A1- 0 756 848        EP-B1- 0 760 091**
**WO-A1-2013/049677        US-A1- 2002 120 203**
**US-B1- 7 139 076**

• **KUENSTNER J. TODD ET AL: "Spectrophotometry of Human Hemoglobin in the near Infrared Region from 1000 to 2500 nm", JOURNAL OF NEAR INFRARED SPECTROSCOPY, [Online] vol. 2, no. 2, 2 March 1994 (1994-03-02), pages 59-65, XP055973079, GB ISSN: 0967-0335, DOI: 10.1255/jnirs.32 Retrieved from the Internet: URL:http://journals.sagepub.com/doi/pdf/10 .12 55/jnirs.32> [retrieved on 2022-10-19]**

(52) Gemeinsame Patentklassifikation (CPC): (Forts.)
G01N 2021/3144; G01N 2201/0231;
G01N 2201/0626; G01N 2201/1211

**Beschreibung**

Es soll ein massenfertigungstauglicher, empfindlicher, ortsauflösender Sensor zur Ermittlung von Substanzmengen in verschiedenen Tiefen von biologischen Geweben und anderen trüben Stoffgemischen anhand ihrer spektralen Signatur geschaffen werden. Die Erfassung soll mit direktem Kontakt zwischen ortsauflösendem Sensor und biologischem Gewebe bzw. Stoffgemisch erfolgen, wobei eine hohe Empfindlichkeit des Sensors gewünscht ist. Eine Herausforderung hierbei ist bei Variation anderer Substanzen die Menge der Zielsubstanz genau zu ermitteln.

Stand der Technik

Die Ermittlung einer Konzentration einer zu bestimmenden Substanz kann über die Absorption der Substanz: $\mu a = c \cdot epsilon$ erfolgen, wobei $\mu a$ der Absorptionskoeffizient, c die Konzentration und epsilon die molare Extinktion sind. Die in der ortsaufgelösten Messung erfasste diffuse Reflexion hängt bei trüben (streuenden) Stoffgemischen jedoch vom Absorptionskoeffizienten $\mu a$ und vom Streukoeffizienten $\mu s$ ab. So ist beispielsweise die gesamte Rückstreuung abhängig vom Verhältnis $\mu s/\mu a$ (aus Streuung und Absorption). Das bedeutet, dass die Messung eines einzelnen Rückstreusignals nicht eindeutig zur Bestimmung des Absorptionskoeffizienten und damit der Konzentration der Zielsubstanz führt. Stattdessen müssen mehrere unabhängige Rückstreusignale ermittelt werden, um eine Trennung von Absorption und Streuung bzw. bei variierendem Streukoeffizienten des Stoffgemisches eine eindeutige Bestimmung der Konzentration einer bestimmten Substanz zu ermöglichen. Diese werden erfindungsgemäß durch die Messung der diffusen Reflexion (Rückstreuung) in mehreren verschiedenen Abständen von einer räumlich begrenzten Strahlungsquelle ermittelt. Durch die Auswertung mehrerer Rückstreusignale lassen sich prinzipiell $\mu s$ und $\mu a$ unabhängig voneinander bestimmen.

Diese Methode an sich ist als "Spatially Resolved Reflectance" (SRR) aus dem Stand der Technik vorbekannt, siehe z.B. WO 2013/049677 A1, EP 0 760 091 B1, EP 0 756 848 B1. Nicht vorbekannt ist dagegen die vorteilhafte Ausgestaltung der hierfür erfindungsgemäßen Vorrichtung und Verfahren. Diese sollen in dieser Anmeldung geschützt werden.

Beschreibung der Erfindung

Der erfindungsgemäße Sensor soll für eine Massenfertigung möglichst derart gestaltet werden, dass eine Übertragung der Kalibrierung mit hoher Genauigkeit von einem vorkalibrierten Sensor auf eine Vielzahl anderer baugleicher Sensoren möglich ist. Dafür sind Aspekte der Sensorgestaltung und der Auswerteverfahren zu berücksichtigen. Zunächst werden für eine hochempfindliche und reproduzierbare Messung die Aspekte der **Sensorgestaltung** beschrieben, dann die **Aspekte der (Mess-)Auswertung** und schließlich die für eine **Massenfertigung** vorteilhafte Ausgestaltung sowie Aspekte der einfachen Übertragung einer Kalibrierung auf einzelne Sensoreinheiten.

Aus der DE 10 2007 054 309 ist bekannt, dass der Abstand d zwischen einem Einstrahlort und einem Detektionsort in einem stark streuenden Medium, d.h. in einem Medium dessen Streukoeffizient $\mu s$ sehr viel größer ist als der Absorptionskoeffizient $\mu a$ sowie das Produkt aus der Dicke d und $\mu s$ sehr viel größer als 1 ist, die Größe des durchstrahlten Volumens beeinflusst. Die Weglänge der Strahlung s nimmt hierbei näherungsweise linear mit dem Abstand d zu (R.A. Bolt, K.R. Rinzema, J.J. ten Bosch, Pure Appl. Opt. 4, 1995). Bei kleiner Absorption beträgt die Verlängerung des Weges $s/d \approx 4{,}5$. Hierdurch werden vor allem diejenigen Anteile der Strahlung erfasst, die aus der Tiefe des Substrats durch Streuung in einiger Entfernung wieder an die Oberfläche gelangen.

Die Strahlungsverteilung kann durch die Diffusionsnäherung der Strahlungstransportgleichung in Abhängigkeit vom Abstand d der Orte für die Einstrahlung und Detektion näherungsweise bestimmt werden (S. Feng, F.-A. Zeng, B. Chance, Appl.Opt. Vol 34 No 19, 1995). Für die Strahlungsausbreitung ergibt sich der Dämpfungskoeffizient $\mu eff$ zu

$$\mu eff = (3\, \mu a\, (\mu a + \mu s(1-g)))^{1/2}$$

wobei $\mu s$ der Streukoeffizient ist und g als mittlerer Kosinus des Streuwinkels die Ausbreitungsrichtung der Strahlung beschreibt.

Die Tiefe, in der das Maximum der detektierten Strahlungsverteilung liegt, kann näherungsweise bestimmt werden zu:

$$zmax = (d/(2 \cdot \mu eff))^{1/2}$$

Erfindungsgemäß ist also die zu ermittelnde Substanz aus Messungen in einer vorbestimmten Tiefe zmax durch Gestaltung des Sensors mit einem aus zmax abgeleiteten Abstand d zwischen Einstrahl- und Detektionsort zu bestimmen. Die Lichtverteilung ist also in dem Zielvolumen, in dem die Konzentration der zu quantifizierenden Substanz ermittelt werden soll, zu maximieren.

Die räumliche Ausdehnung des Messvolumens erstreckt sich als bananenförmiges Volumen von der Oberfläche des Messgegenstands von Einstrahlort zu Detektionsort, so dass für eine zuverlässige Quantifizierung auch die Anteile in den über dem Zielvolumen liegenden Schichten erfasst werden müssen, damit diese herausgerechnet werden können. Die maximale Tiefe der Lichtausbreitung liegt bei homogenen Medien etwa in der Mitte zwischen Einstrahlort und Detektionsort.

Der erfindungsgemäße Sensor hat also mehrere Strahlungsquellen und mehrere Detektoren in unterschiedlichen Abständen d voneinander, die verschiedene Messwerte liefern, die die Erfassung unterschiedlicher Zielvolumina ermöglichen, wobei sich die Zielvolumina zumindest teilweise überlappen.

Eine vorteilhafte Ergänzung der Alternative zur Bestimmung der durchstrahlten Volumina stellt die Simulation der Strahlungsausbreitung im Messgegenstand dar, z.B. durch die vorbekannte Monte-Carlo-Simulation. Hierfür müssen allerdings Annahmen über die im Messgegenstand vorherrschenden optischen Eigenschaften $\mu a$, $\mu s$ und $g$ gemacht werden. Die Annahmen von $\mu s$ und $g$ können entsprechend der Formel $\mu s' = \mu s \cdot (1-g)$ auch durch Annahme des reduzierten Streukoeffizienten $\mu s'$ ersetzt werden.

Die Festlegung der Lichtverteilung über die Abstände zwischen Strahlungsquelle und Detektor(en) (Einstrahl- und Detektionsort(e)) kann einer bevorzugten Ausführungsvariante gemäß zur Tiefenwichtung der Konzentrationsbestimmung genutzt werden, etwa um in Schichtsystemen für jede Schicht Konzentrationen zu bestimmen oder Oberflächeneffekte bzw. Effekte durch tiefere Schichten zu korrigieren.

Vorzugsweise wird die gewünschte bzw. erforderliche Weglänge im Gewebe gewählt, indem abhängig von der maximal zulässigen Einstrahlungsintensität die minimal mit dem Detektor erfassbare Signalhöhe, die proportional zu $e^{-\text{Faktor} * \text{Weglänge} * \text{Absorption bei der gewählten Wellenlänge}}$ ist, dahingehend optimiert wird, dass die Weglänge für lange Wege eine empfindliche Detektion, aber auch ein kleines Signal bewirkt. Dabei steht der Faktor für die Lichtwegverlängerung im Messgegenstand, Faktor * Weglänge gibt damit die optische Pfadlänge an. Es gilt also für die durch die Zielsubstanz bestimmten Wellenlängen (siehe weiter unten zur Auswahl der Wellenlängen) die Absorption des Messgegenstandes zu ermitteln sowie die minimale Signalhöhe am Detektor zu ermitteln, so dass eine möglichst lange Weglänge genutzt werden kann für den größten Abstand d zwischen Strahlungsquelle und Detektor.

In homogenen Medien ist eine Berücksichtigung der dabei erreichten Messtiefe nicht wichtig, bei Schichtsystemen ist die Lage des Zielvolumens auch ein begrenzender Umstand.

Die Anordnung der Detektoren kann mit gleichem Abstand oder ebenso mit unterschiedlichem Abstand zueinander erfolgen. Eine besonders vorteilhafte Anordnung ist gegeben, wenn die Detektoren sich in zunehmendem Abstand voneinander mit zunehmender Entfernung von der Strahlungsquelle befinden. Diese Anordnung der Detektoren zueinander muss nicht entlang einer Linie sein, sondern kann beliebig sein, auch ring- oder sternförmig.

Lange Strahlungswege in absorbierenden bzw. streuenden Messgegenständen bedingen eine Abnahme der am Detektionsort auf den Detektor auftreffenden Strahlungsmengen. Somit ist eine Vergrößerung des detektierten Signals durch Einsatz mehrerer Detektoren mit gleichem Abstand vom Einstrahlort, die beliebig angeordnet sein können, oder auch die Vergrößerung der Detektorfläche im Sensor vorteilhaft. Dies schließt den Einsatz ringförmiger Detektoren ein, ebenso wie die Nutzung mehrerer Bauteile, die nebeneinander angeordnet sind.

In vorteilhafter Weise ist die Gestaltung des Sensors derart, dass die Lichtanteile auf die in unterschiedlichen Abständen angeordneten Detektoren durch eine unterschiedliche Verstärkung auf gleiche Signalamplituden angehoben werden. Insbesondere erfolgt die Anpassung der Verstärkung an die durch Test oder Simulation am Messgegenstand zu erwartenden Strahlungsmengen bevorzugt je Detektor.

Die Auswahl der Wellenlängen erfolgt mit Hinblick auf die Zielsubstanz, bzw. Zielsubstanzen vorzugsweise derart, dass mindestens je eine Wellenlänge oder ein Wellenlängenbereich benutzt wird, in dem für die zu ermittelnde Zielsubstanz die Sensorsignale eindeutige Auswirkungen auf die von dem bzw. den Detektoren erfassten Strahlungsmengen zeigen. Weiterhin ist zur Erfassung von Störsubstanzen oder anderen Störeinflüssen mindestens eine Wellenlänge oder ein Wellenlängenbereich einzustrahlen, in dem eine oder mehrere Zielsubstanzen sehr geringe bis keine Auswirkungen auf die Sensorsignale, d.h. die von dem bzw. den Detektoren erfassten Strahlungsmengen zeigen. Somit ist vorzugsweise immer mindestens ein Paar von Auswirkung-zeigender und keine Auswirkung zeigender Wellenlänge der Strahlung im Abstand d, der das Zielvolumen determiniert, einzustrahlen. Die keine Auswirkung auf die Sensorsignale zeigenden Wellenlängen können auch für mehrere Zielsubstanzen gleich sein und dienen der Störungsunterdrückung. Die Nutzung von mehr als einer eine Auswirkung auf die Sensorsignale zeigenden Wellenlänge je Zielsubstanz erhöht vorteilhaft die Genauigkeit und damit die kleinste quantifizierbare Konzentration der Zielsubstanz im Zielvolumen.

Diese Wellenlängen können sequentiell eingestrahlt werden und gleichzeitig von breitbandigen Detektoren in verschiedenen Abständen d empfangen werden.

Die weiter unten beschriebene Lock-In-Technik kann auch benutzt werden, um jede Wellenlänge einzeln mit einer unterschiedlichen Frequenz zu beaufschlagen und so die LED kontinuierlich (nicht sequentiell) zu betreiben und mit breitbandigen Detektoren durch spektrale Frequenzanalyse oder durch sequentielle Demodulation mit der jeweiligen Frequenz die einzelne Wellenlänge wieder herauszufiltern.

Außerdem ist die Nutzung von spektral auflösenden Detektoren vorteilhaft, die eine gleichzeitige Einstrahlung von vielen Wellenlängen, z. B. einer Weißlichtquelle wie einer Glühlampe oder einer Weißlicht-LED, ermöglichen.

Der Einsatz eines die Strahlungsemission der Strahlungsquelle ohne Interaktion mit anderen Stoffen detektierenden Bauteiles (Referenzdetektor) ist ebenfalls vorteilhaft. Dieser Referenzdetektor kann in unmittelbarer Nähe der Strahlungsquelle platziert sein aber auch über Strahlungsanteile umlenkende Bauteile die Intensität der Strahlungsquelle erfassen.

Um die Temperaturabhängigkeit der Strahlungsquellen sowie Mess- und Referenzdetektoren korrigieren zu können, ist es vorteilhaft einen Teil der Strahlungsemission der Strahlungsquelle auf den Referenzdetektor zu leiten und so zu erfassen.

Um den Einfluss der Temperatur auf diese durch Reflexion realisierte Übertragung zu minimieren, wurde die Temperaturabhängigkeit der Reflektivität unter 90° von unterschiedlichen Oberflächen untersucht.

| Material/ Bauteil | Oberflächenbearbeitung | Änderung der Reflektivität bei $\Delta T = 25$ K in % Remission |
|---|---|---|
| POM schwarz | gefräst | 2,9 |
| POM weiß | gefräst | 0,7 |
| PTFE weiß | gedreht | 0,6 |
| Spectralon/ Reflexionsstandard | | 0,0 |
| Aluminiumlegierung | gefräst | 0,4 |
| Aluminiumlegierung | gefräst, schwarz eloxiert | 1,7 |
| Aluminiumlegierung des Sensorträger | gefräst, schwarz eloxiert | 15,5 |
| AlN-Keramik | | 0,0 |

*Änderung der Reflexivität bei Erwärmung von 20°C um 25 K*

Vorteilhafterweise sollte also Spectralon, AlN-Keramik oder blanke, gefräste Aluminiumlegierung für die Strahlungsumlenkung zum Referenzdetektor genutzt werden.

Um die Strahlungsabgabe der Strahlungsquelle zu stabilisieren ist der Einsatz einer Konstantstromquelle oder stabilisierten Spannungsversorgung vorteilhaft. In Weiterführung des Erfindungsgedankens ist der Einsatz einer Lock-In-Technik mit gepulst oder sinusförmig abgegebener Intensität und entsprechend in dieser an die Störungen wie weiter unten beschrieben angepassten Frequenz bzw. mit höherer Abtastung ausgelesenen Detektoren möglich.

Eine sehr genaue Einhaltung exakt definierter Abstände zwischen Strahlungsquelle und Detektoren) ist nicht erforderlich, sondern kleinere Abweichungen und Ausdehnungen im Rahmen der üblichen Platzierungsgenauigkeit bei kleinen Bauteilgrößen z. B. von als Chip, d.h. gehäuseloses Bauteil, montierten Bauteilen sind durch die Beherrschung der restlichen vorteilhaften Maßnahmen bei stark streuenden Messgegenständen ohne großen Einfluss auf die genaue Ermittlung der Mengen der Zielsubstanz. Die zulässigen kleineren Abweichungen, d.h. die Toleranz steht in einem linearen Zusammenhang zu dem Abstand x in dem die Strahlung auf 1/e abgefallen ist und zu der gewünschten Genauigkeit der Bestimmung der Zielsubstanz; die Toleranz soll kleiner als x/n sein, wobei n durch die gewünschte Genauigkeit der Konzentration der Zielsubstanz festgelegt ist.

Die Nutzung gehäuseloser Bauteile für die Strahlungsquellen ermöglicht eine kompakte Platzierung mehrerer Quellen auf einer kleinen Fläche, um ohne strahlungsformende Elemente einen einzigen Einstrahlort für mehrere frei wählbare Wellenlängen auf dem Messgegenstand zu realisieren. Ebenfalls wird durch gehäuselose Bauteile als Strahlungsquellen eine Platzierung mit geringem Abstand und Einbringung gleichförmiger, überlappender Lichtverteilung auf dem Messgegenstand (Einstrahlort) ermöglicht sowie für die Detektion die Erfassung der Lichtverteilung mit hoher Ortsauflösung ermöglicht.

Auch die Nutzung von kleinen gehausten Bauteilen, die z.B. Gruppen von LEDs oder Photodioden oder beides bzw. ähnliche Strahlungsquellen und / oder Detektoren enthält, ist bevorzugt.

Somit ist eine Sensorgestaltung mit nebeneinander angeordneten Beleuchtungsbauteilen, d.h. Strahlungsquellen, und davon im Abstand d (beispielsweise bezogen auf die Bauteilmittelpunkte) angeordneten Detektorbauteilen, d.h. Strahlungsdetektoren, möglich und bevorzugt.

Ebenfalls vorteilhaft und bevorzugt ist die Nutzung gleichartiger Bauteile für die Detektoren bei allen Abständen.

Dann sind die Bauteileigenschaften, wie z.B. Temperaturabhängigkeiten, gleichartig und somit einfacher korrigierbar.

In Weiterführung des Erfindungsgedankens ist der Einsatz von nicht-linearen, bzw. logarithmischen Verstärkervorrichtungen bevorzugt. Diese nicht-linearen, bzw. logarithmischen Verstärkervorrichtungen können die an den Detektoren erzeugten Signale durch höhere Verstärkung der schwächeren Signale in Bezug auf die näher am Einstrahlort liegenden Detektoren auf gleiche Signalamplituden bringen, was vorteilhaft für die Weiterverarbeitung, Digitalisierung, bzw. Auswertung ist.

In Weiterführung des Erfindungsgedankens wird der Sensor als Modul, das wie ein elektronisches Bauteil in Vorrichtungen eingebaut werden kann, mit mindestens einer LED und mindestens einer Photodiode in einem vergossenen Gehäuse gefertigt. Ebenfalls vorteilhaft ist es die Ansteuerungs-Elektronik und die Vorverstärker in das Modul zu integrieren. Ebenfalls können auch, jeweils nur LED bzw. nur Photodioden in einem Modul für den Sensor genutzt werden.

Weiterhin vorteilhaft ist die Korrektur der Temperaturabhängigkeit von Strahlungsquelle und Detektor mit einer bekannten Temperaturcharakteristik oder einer Temperaturmessung.

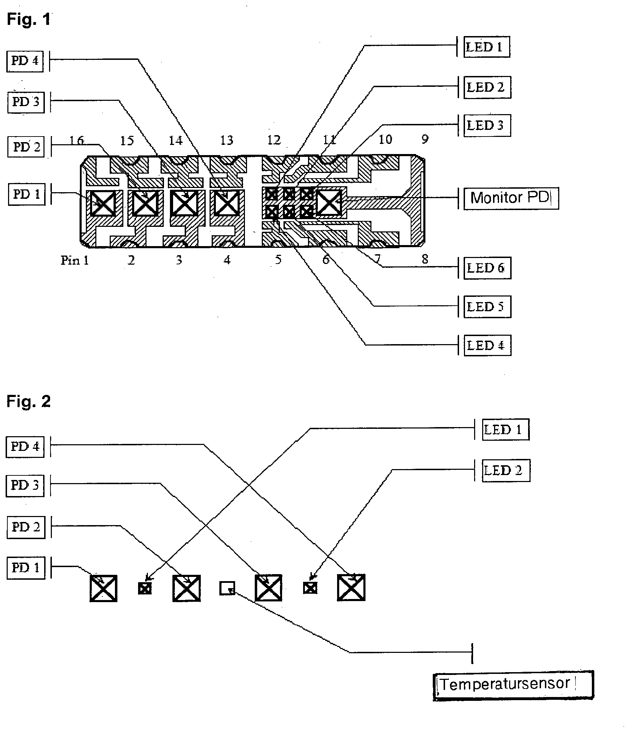

**Fig. 1** zeigt eine exemplarische Ausführungsform, in der vier Photodioden (PD), sowie sechs LED als Strahlungsquellen und eine Monitoring-Photodiode als Referenzdetektor, mit zueinander spezifischen Abständen eingegossen sind und deren Kontakte nach außen geführt sind, wobei die Bezeichnungen LED auch für eine andere Strahlungsquelle und PD auch für einen anderen Detektor stehen kann. Die Zahlen bezeichnen die durchgezählten nach außen geführten Anschlüsse (Pins), die Bauteile sind mittels Bonding oder ähnlichen Techniken gemäß dem Stand der Technik elektrisch verbunden. Ohne Beschränkung der Erfindung können die Abstände und Wellenlängen bzw. die allgemeine äußere Bauform des Moduls, die Form und der Ort von Strahlungsbarrieren und weiter in diesem Dokument genannte Aspekte entsprechend den in diesem Dokument genannten Gestaltungsrichtlinien angepasst bzw. dem Stand der Technik entsprechend verändert werden.

In Weiterführung des Erfindungsgedankens ist es ebenfalls möglich, Module oder Baugruppen tauschbar mit Schnittstellen zu gestalten, so dass beim Aufbau einer Vorrichtung diese einfach ausgetauscht werden können. Dies ermöglicht sowohl den Ersatz defekter Baugruppen, wie auch die einfache Anpassung an verschiedene Zielvolumina (Messtiefe) durch Austausch von Detektorgruppen mit anderen Abständen, wie auch anderer Zielsubstanzen oder auch anderes Medium durch Austausch der Baugruppen mit der bzw. den Strahlungsquelle(n).

In Weiterführung des Erfindungsgedankens ist ebenfalls eine Mischung der Abfolge von Photodioden und Strahlungsquellen möglich, so dass Strahlungsquellen zu einem benachbarten Detektor einen kurzen Abstand, zu einem anderen einen größeren Abstand aufweisen. Neben dem für die erste Strahlungsquelle in größerem Abstand platzierten Detektor kann eine weitere Strahlungsquelle platziert sein, die in Vertauschung der Detektoren für die Auswertung ähnliche Abstände aufweist und so eine zweite Wellenlänge mit in umgekehrter Reihung gleichen Abständen darstellt, wobei die Detektoren bei der Auswertung ebenfalls in umgekehrter Reihung zugeordnet werden. Diese verzahnte Anordnung ermöglicht einen platzsparenden Aufbau eines solchen Sensors. Exemplarisch könnte die Anordnung wie in **Fig. 2** aufgebaut sein. Ohne Beschränkung der Erfindung sind andere, auch asymmetrische Anordnungen, auch Anordnungen mit einer anderen Anzahl von Bauteilen bzw. ohne Temperatursensor erfindungsgemäß.

Der Aufbau des Sensors kann ebenfalls in Weiterführung des Erfindungsgedankens verändert werden, indem eine Trennung des Ortes des Strahlaus- und-eintrittes vom Ort der Bauteile erfolgt. So kann bevorzugt durch eine Übertragungsoptik zwischen Strahlungsquelle und Austrittsfenster z.B. durch eine Glasfaser oder einen Glasstab mit mindestens einem Prisma oder ähnliche Strahlfortleitungen entsprechend dem Stand der Technik der Sensoraufbau geometrisch entkoppelt werden, während er optisch durch die Strahlfortleitungen gekoppelt bleibt.

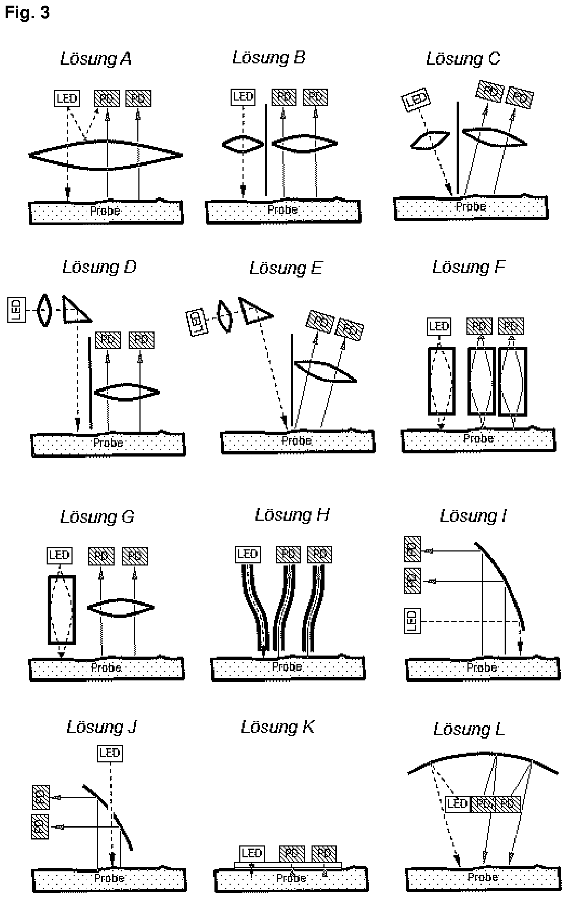

In Fortführung des Erfindungsgedankens kann der Sensor in direktem Kontakt zum Messgegenstand messen, oder es ist eine rein optische Ankopplung an den Messgegenstand durch Abbildung von Lichtquellen und Detektoren auf den Messgegenstand durch Linsensysteme oder ähnliche Vorrichtungen oder eine mechanische und optische Ankopplung an den Messgegenstand vorgesehen. Dies sei im Weiteren anhand **Fig. 3** ausgeführt, wobei die Bezeichnungen LED für eine Strahlungsquelle (auch andere als LED) und PD für einen Detektor (auch andere als eine Photodiode) stehen können:

Grundsätzlich stellt sich für den erfindungsgemäßen Sensor das Problem, mit einer Strahlungsquelle eine in gewissem Abstand befindliche Probenoberfläche lokal zu beleuchten sowie ortsaufgelöst die abstandsabhängige Remission zu detektieren. Hierbei ist es wünschenswert eine ausreichende Schärfentiefe der Abbildung zu erreichen, die aber mit dem erreichbaren Lichtdurchsatz (der durch die NA (numerische Apertur) bestimmt wird) in einem reziproken Verhältnis steht (NA$\uparrow$ $\Rightarrow$ Schärfentiefe$\downarrow$). Vorteilhaft ist die Nutzung eines Abstandshalters, der es ermöglicht den Abstand zwischen Sensor und Messgegenstand genau einzuhalten. Ebenfalls vorteilhaft ist eine andere Methode zum Bestimmen des Abstandes, der dann vorteilhaft auch durch automatisierte Verfahren konstant gehalten werden kann.

Eine der möglichen Lösungen ist eine gemeinsame Sammellinse für Strahlungsquelle und Detektoren (**Fig. 3** Lösung A): Diese Lösung ist nicht praktikabel, da die Photodioden über die Reflektion an der Glasoberfläche direkt bestrahlt würden.

Nutzt man zwei getrennte Sammellinsen (**Fig. 3** Lösung B) mit dazwischen platzierter Strahlungsbarriere, so erreicht man zwar eine gemeinsame Abbildung, aber die Baugröße ist recht groß. Benötigt man kleine Abstände (dies sind die mit dem Vergrößerungs- bzw. Verkleinerungsverhältnis multiplizierten Ortsauflösungen), so ergibt sich der Bedarf an kleinen Linsendurchmessern, die nur eine kleine NA haben.

Um diese Problematik zu entschärfen kann man mit 2 getrennten Sammellinsen die Strahlwege gegeneinander verkippen (**Fig. 3** Lösung C). Dies führt zu der Problematik, dass die Messung im vorgegebenen Abstand von Beleuchtung zu den Detektionsorten nur mit dem korrekten Abstand des Sensors zur Probenoberfläche erfolgen kann, da der Abstand der Beleuchtungs- und Detektionsspots vom Abstand zur Oberfläche abhängig ist, also für die Auswertung evtl. nicht nachvollziehbar. Zudem ergibt sich aus der Kippung ein mögliches Problem mit der Abbildungsebene, die geneigt zu der Ebene der Photodioden steht. Hierzu wird also eine größere Schärfentiefe oder ein spezielles Kippen einer der Platinen gefordert.

Nutzt man einen Spiegel oder ein Prisma, um einen der Strahlenwege um 90° abzuwinkeln (**Fig. 3** Lösung D), so rücken die Beleuchtungs- und Detektionsspots zwar nah aneinander, es gibt aber immer noch einen relativ großen Abstand (einige mm) zwischen Beleuchtung und Detektion in Höhe der Linsenebene.

Zusätzlich könnte man die Strahlwege gegeneinander kippen (**Fig. 3** Lösung E), was aber ebenfalls die bei **Fig. 3** Lösung C genannten Probleme mit sich bringt.

Grundsätzlich anders sieht es aus, wenn man GRIN-Linsen nutzt (**Fig. 3** Lösung F). Damit ist man jedoch auf einige wenige mm Abstand von der Linsenoberfläche zur Probe beschränkt, da die GRIN-Linsen nur kleine Brennweiten haben. Die optische Auslegung ist zudem sehr genau auszuführen, da eine Anpassung durch Längenänderung der GRIN-Linsen erfolgt. Damit ergeben sich höhere Anforderungen an die Herstellung der passenden GRIN-Linsen, die auf Länge geschliffen und anschließend poliert werden müssen. Allerdings sind GRIN-Linsen mit typischerweise großer NA geeignet, um vorteilhaft einen großen Anteil remittierten Lichts einzusammeln.

Auch eine Kombination von GRIN- und Sammellinse (**Fig. 3** Lösung G), d.h. die Beleuchtung durch die GRIN-Linse zu führen, hat noch die Beschränkung der Nähe zur Probenoberfläche und aufwändigen Herstellung.

Die Nutzung von dickeren Fasern, also Glasstäben, die auch gerundete Endflächen haben könnten (**Fig. 3** Lösung H), würde nur eine Zusammenführung der optischen Aus- bzw. Eintrittsbereiche bei gleichzeitig gespreizter Anordnung der LED oder Photodioden erbringen, die NA bleibt klein und der Lichtdurchsatz auch. Zudem ist eine Herstellung solcher speziell geformter Glasstäbe aufwändig.

Die Nutzung eines Hohlspiegels (**Fig. 3** Lösung I), der off-axis, also gekippt und seitlich beleuchtet genutzt werden könnte ist erfindungsgemäß. Die Beleuchtungs-LED darf den Spiegel nicht überstrahlen, die NA ist auf etwa 0,2 begrenzt, aber aufgrund ungenügender Abbildungsqualität bleibt diese Anwendung auf größere Abstände Lichtquelle / Detektionsort begrenzt. Es gibt keinen Reflex der LED von der Spiegeloberfläche auf die Photodioden, da nur Streuung dies hervorrufen könnte. Die Hohlspiegeloberfläche muss also sehr gut poliert sein und darf keinen Staub anhaften haben.

Weitergedacht kann der Hohlspiegel auch mit einem Loch versehen sein, durch das die LED abstrahlt und das als Blende wirkt (**Fig. 3** Lösung J). Hier muss. eine zusätzliche Abbildungsoptik für die LED vorgesehen werden.

Allerdings ist bei allen bisherigen Lösungen die direkte Reflexion der Beleuchtung auf der Probenoberfläche in die Photodioden nicht ausgeschlossen. Dies ist bei der optik-losen Variante, also 2 getrennte Fenster mit Strahlungsbarriere in Kontakt mit der Probe (**Fig. 3** Lösung K), gegeben, wo auch die NA von nahezu 1 den maximal realisierbaren Zustand darstellt. Ebenfalls ist dies möglich bei getrennten Optiken (GRIN, Fasern, Stäbe, Linsen), wenn eine Strahlungsbarriere eingebaut werden kann.

Erfindungsgemäß ist ebenfalls das Ziel einer kontaktlosen Messung mit einem Mindestabstand. In Weiterführung des Erfindungsgedankens kann ein senkrecht zur Probenoberfläche ausgerichteter Hohlspiegel genutzt werden, in dem in Richtung der optischen Achse mittels Draht oder einer ähnlichen, nicht stark strahlungsabsorbierenden Vorrichtung eine Platine gehaltert ist (**Fig. 3** Lösung L), die LED und Photodioden trägt. Dabei sind die Ränder so geschwärzt, dass kein LED-Licht rückreflektiert wird oder überstrahlt.

In Weiterführung des Erfindungsgedankens ist ebenfalls erfindungsgemäß eine Halterung des Messgegenstandes mit zu diesem definiert angeordnetem Strahlaus- und-eintritt. Dies kann eine Halterung für ein schlauchgeführtes Medium sein, dessen Innendurchmesser mit dem Außendurchmesser des Schlauches gut übereinstimmt. Erfindungsgemäß könnte eine parallele Strahlungsführung (Referenzzweig) ohne Medium für eine Korrektur genutzt werden - für diese Möglichkeit der Korrektur siehe weiter hinten die Ausführungen zum Messablauf. Vorteilhaft ist es, die parallele Strahlführung so auszulegen, dass ein anderer Schlauchdurchmesser dort gehaltert werden kann und durch Änderung der Auswertung, d.h. Vertauschung der Referenzzweige, so zwei Schlauchdurchmesser mit idealer optischer Ankopplung gemessen werden können. Die parallele Strahlführung kann einen leeren Schlauch oder keinen Schlauch enthalten. Die Korrektur erfolgt jeweils anhand der im Referenzzweig erhobenen Werte. Diese können auch modifiziert in die Korrektur eingehen.

Ausgehend von einem derart gestalteten Sensor ist eine Strahlungsbarriere zwischen Strahlungsquelle und Strahlungsdetektor als Maßnahme zur Erhöhung der Genauigkeit vorteilhaft. Die Nutzung einer Abgrenzung der Strahlungsquelle und ggf. der Detektoren an der Oberfläche zum Messgegenstand ermöglicht erst die genaue Definition der Lichtwege und damit der Abstände d. Da bei Nutzung von gehäuselosen Bauteilen ein Kontakt der Bauteile mit dem Messgegenstand zu Beschädigungen der Verbindungselemente führt, z. B. durch Abreißen von Bond-Drähten oder Schäden durch punktuelle mechanische Krafteinwirkung, ist eine Messung in Kontakt nur mittels eines Abstandhalters oder durch eine Schutzabdeckung hindurch möglich. Ebenso erfordern Aspekte der Sensoranwendung, z. B. die Möglichkeit einer Reinigung, eine Schutzabdeckung. Eine solche Schutzabdeckung kann aus einem in einem kurzen Abstand vom Bauteil angeordneten Schutzfenster aus Kunststoff oder Glas sowie aus einem Auffüllen der überstehenden Bauteil- oder Verbindungselemente mit einer für die Strahlung transparenten Vergussmasse bestehen. Eine Fortleitung von Strahlung zwischen den Grenzflächen eines transparenten Schutzfensters oder eine Reflexion an der Oberfläche einer für die Strahlung transparenten Vergussmasse kann nicht verhindert werden.

Die Strahlungsausbreitung vom Bauteil zum auf dem Messgegenstand befindlichen Einstrahlort ist somit nicht unmittelbar und optisches Übersprechen ist möglich. Vorzugsweise wird die ungewollte, da nicht signaltragende Einstrahlung von der Strahlungsquelle auf den bzw. die Detektoren ohne Durchgang durch den Messgegenstand durch eine Strahlungsbarriere, die auch eine Trennung der Schutzabdeckung beinhaltet, verhindert. Diese Strahlungsbarriere muss eine ausreichend hohe Dämpfung, d.h. Dicke > ($3/\mu a$ des Strahlungsbarrierenmaterials), aufweisen und dient zum einen der Reduktion von optischem Übersprechen und zum anderen der Reduktion von Hintergrundlicht / Fremdlicht / Störlicht, welche zusätzliches Rauschen im Detektor verursachen. Zusätzlich kann auch eine weitere Strahlungsbarriere um die Strahlungsquellen und Detektoren vorgesehen werden. Weiterhin sind Strahlungsbarrieren vorteilhaft, die für jeden Detektor die erfasste Oberfläche des Messgegenstands eingrenzen, so dass der Abstand zwischen Einstrahlort und Detektionsort genauer festgelegt werden kann und insbesondere Strahlungsanteile, die nur unter der Strahlungsbarriere innerhalb des Messgegenstands gestreut werden und schräg auf einen weiter entfernten bzw. näheren Detektor treffen, sozusagen "falsch" einem Abstand d zugeordnet werden.

Zum anderen wird durch die Barriere der Einfluss von durch die Sensoranwendung hervorgerufener Varianz des optischen Übersprechens durch Handhabung oder unterschiedliche Umgebungen reduziert.

Die Trennung der Schutzabdeckung hat einen ähnlichen Effekt. Die Weiterleitung durch Reflexion an einer oder beiden Grenzflächen einer flächenhaft ausgebildeten Schutzabdeckung wird am Ende der Schutzabdeckung ausgekoppelt und kann nicht mehr in einen Detektor fallen. Somit ist vorzugsweise die Trennung der Schutzabdeckung zwischen Einstrahlungs- und Detektionsseite sowie in Weiterführung des Erfindungsgedankens auch die Trennung der Schutzabdeckung für jeden der Detektionsabstände vorgehen.

Die Strahlungsbarriere ist vorzugsweise so gestaltet, dass sowohl die direkte Bestrahlung über eine Schutzabdeckung wie auch die Strahlungsübertragung insbesondere über das die Bauteile tragende Substrat sowie die umgebenden Gehäusebestandteile vermieden wird. Dies kann entsprechend dem Stand der Technik durch für die genutzten Wellenlängen stark absorbierende Beschichtungen (Lacke, Metallbeschichtung, etc.) wie auch durch die Strahlung stark absorbierende Blenden oder geeignet angeordnete die Strahlung stark absorbierende Vergussmassen erfolgen. Eine mögliche Ausführung für solche Vergussmassen ergibt sich bei Nutzung einer transparenten Vergussmasse auf dem Sensor, wobei Schlitze oder Gräben in die transparente Vergussmasse bis zum die Bauteile tragenden Substrat eingebracht werden, die mit nichttransparenter Vergussmasse verfüllt werden.

Eine andere Ausführung für solche Vergussmassen wäre die Verfüllung von Hohlräumen im Sensorgehäuse, die nur einen direkten Bereich der Strahlungsausbreitung von der Strahlungsquelle zur Oberfläche des Messgegenstands und von den in Abstand d zu erfassenden Orten auf der Oberfläche des Messgegenstands zu dem bzw. den Detektoren aussparen. In Erweiterung des Erfindungsgedankens können solche Aussparungen in der absorbierenden Vergussmasse durch eine vorher aufgebrachte, geometrisch vorbestimmte, transparente Vergussmasse oder andere für die Strahlung transparente Vorrichtungen erzeugt werden.

Ebenfalls kann im Falle des die Bauteile tragenden Substrats auch die mechanische Trennung der die Strahlungsquellen tragenden und der die Detektoren tragenden Substratteile erfolgen.

Eine weitere vorteilhafte Lösung ist es, die Strahlungsbarriere aus der an den Messgegenstand zu bringenden Oberfläche leicht vorstehen zu lassen, beispielsweise, indem die Strahlungsbarriere über eine vorgesehene Kontaktfläche mit dem Messgegenstand zwischen 0,1 mm und 1 mm hinausragt. Kann diese überstehende Strahlungsbarriere in die Oberfläche des (weichen) Messgegenstands eingedrückt werden oder ist die Barriere deformierbar, wird hierdurch das durch einen Luftspalt hervorgerufene optische Übersprechen, d.h. der Strahlungsübertritt aus der Strahlungsquelle in den oder die Detektoren ohne durch das Messgegenstand zu treten, in überraschender Weise wirksam unterbunden. Ist der Messgegenstand nicht weich genug, so bildet sich auf einer Seite ein größerer Luftspalt (als ohne vorstehende Barriere), der jedoch von der Strahlungsbarriere begrenzt wird. Auch hier ist ein optisches Übersprechen unterbunden.

Eine weitere Gestaltungsgröße ist die thermische Beeinflussung der Bauteilcharakteristika bzw. des Messgegenstands. In erster Linie ist also eine Nutzung möglichst temperaturunabhängiger Bauteile vorteilhaft.

Ändert der Messgegenstand mit der Temperatur seine optischen Eigenschaften, so ist dessen Temperatur an der Kontaktfläche mit dem Sensor zu erfassen und in die Auswertung mit einzubeziehen.

Auch jede Veränderung der Bauteileigenschaften durch die Temperatur ist problematisch. Es ist bekannt, dass im Falle von LED als Strahlungsquellen im Strombetrieb unter Temperaturerhöhung die abgestrahlte Leistung vermindert wird. Ein Einfluss der Temperaturerhöhung auf die Detektoren ist ebenfalls gegeben, so wurde bei einer Silizium-Photodiode für Wellenlängen < 500 nm mit Zunahme der Temperatur von Raumtemperatur auf ca. 45°C eine zunehmende Empfindlichkeit beobachtet, d.h. Signalerhöhung bei gleicher Strahlungsmenge. Bei anderen Wellenlängen bzw. Bauteilen bzw. Temperaturbereichen zeigen sich davon verschiedene Änderungen. Ebenfalls ist bekannt, dass bei zunehmender Temperatur die elektronische Verstärkung das Signal mit erhöhtem Rauschen beaufschlägt. Die Beeinflussung der Bauteile durch die Temperatur führt zu unterschiedlichen Genauigkeiten in der Quantifizierung, wenn die jeweiligen Temperaturen der Bauteile nicht kontrolliert werden oder bekannt sind und deren Einfluss bzw. Einflüsse korrigiert werden.

Diese Wärmeeinflüsse können durch Erfassung der Temperatur der Bauteile bzw. der Temperatur in der Nähe der Bauteile entsprechend vorab erfolgter Messungen korrigiert werden. Ebenfalls können die Korrekturen anhand von Datenblättern/Herstellerangaben erfolgen. Die Korrektur erfolgt nach der Erfassung der Sensorwerte im Rahmen der Auswertung des verstärkten Signals oder elektronisch als analogtechnische Anpassung der Gesamtverstärkung zu einer linearen Kennlinie mit sich mit der Temperatur gegenläufig zur Temperatur-Kennlinie der zu korrigierenden Bauteile ändernden Bauteilen im Rückkopplungszweig der Verstärker oder parallel zum zu korrigierenden Bauteil. Ebenfalls vorteilhaft ist ein ansteuerbares Bauteil, welches die aktuelle Verstärkung anhand der Signalwerte anpasst, z.B. ein durch eine Rechenvorrichtung gesteuertes digitales Potentiometer. Eine solche Temperaturerfassung findet vorzugsweise auf dem Substrat in der Nähe der Bauteile statt.

Vorzugsweise sind ebenfalls eine thermische Abgrenzung der Strahlungsquellen von den Detektoren, sowie die geeignete Materialwahl für das die Bauteile tragende Substrat vorgesehen, damit sich die Bauteile durch hohe Wärmeleitung weitgehend auf dem gleichen Temperaturniveau befinden. Eine Wärmeleitung ausgehend von den Strahlungsquellen als primäre Wärmequellen im Sensor ist nicht sehr vorteilhaft, da dies über den Sensor gesehen, d.h. an den in verschiedenem Abstand d positionierten Detektoren, immer einen Temperaturgradienten erzeugen würde.

Ebenfalls bevorzugt ist die Nutzung von Bauteilen zur Erhöhung der Temperatur auf ein stabilisiertes Niveau, welches entweder über dem im normalen Betrieb erreichten Niveau liegt oder bei dem die im Betrieb auftretenden Änderungen der Bauteileigenschaften ausreichend klein sind um eine ausreichend hohe Genauigkeit der Signale zu gewährleisten, die der aufzulösenden Änderung der Stoffmenge entspricht. Die Temperatur ist zu erfassen und durch geregelte Wärmezufuhr auf einen Sollwert zu stabilisieren.

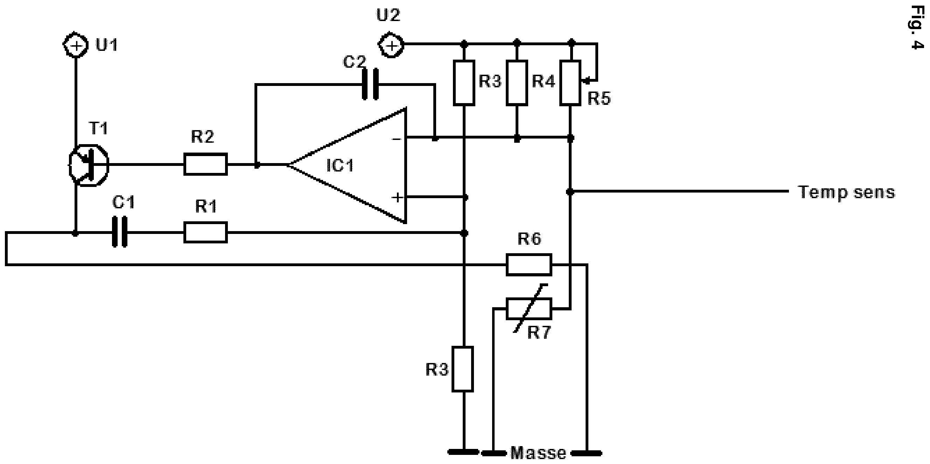

In **Fig. 4** ist eine exemplarische Lösung der Temperatur-Stabilisierung eines Bauteile tragenden Substrates dargestellt. Die Schaltung heizt über den Widerstand R6, der thermisch mit dem temperaturabhängigen Widerstand R7 gekoppelt ist, die Strahlungsquellen. Die Einstellung der Zeitkonstanten für die Regelung erfolgt über die Auslegung von R1 und C1. Die damit eingestellte Zeitkonstante sollte vorteilhaft der Zeitkonstante der thermischen Kopplung zwischen R6 und R7 entsprechen, damit der Regelkreis stabil regeln kann. C2 dient der Unterdrückung der Schwingungsneigung des Regelkreises. Die Widerstände R3 teilen die Spannung auf die Hälfte der Spannung U2. Falls eine Spannungsquelle für mehrere elektronische Schaltungen vorgesehen ist, kann U2 hier aus dieser Spannungsquelle gespeist werden. Ist keine weitere Spannungsquelle vorhanden, kann U2 auch mit U1 identisch sein. Von U1 fließt der Strom zum Heizen des Widerstandes R6, wodurch hier ein eigener Stromkreis vorteilhaft ist.

Die Brückenschaltung wird mit dem durch die Widerstände R3 und R3 gebildeten temperaturstabilen Zweig und dem durch die Widerstände R4 und R5 sowie R7 gebildeten temperaturabhängigen Zweig auf Spannungsgleichheit in der Brücke geregelt. IC1 verstärkt die in der Brückenschaltung auftretenden Abweichungen und steuert den Transistor T1 an, welcher den Heizwiderstand R6 mit Strom beaufschlagt, bis der Widerstand R7 über die Änderung aufgrund der thermischen Rückkopplung über den IC1 dies unterbindet.

Das System ist rein analog, ohne digitale Steuerung, in der Lage eine über die Widerstände R4 und R5 voreinstellbare Solltemperatur aufrecht zu erhalten. R4 dient nur der Anpassung der mit R5 einstellbaren Werte und kann auch entfallen. Wird der Spannungswert, welcher an Temp sens anliegt, ausgewertet so kann darüber die an R7 anliegende Temperatur ermittelt werden.

Ebenfalls bevorzugt ist eine thermisch gut leitfähige Verbindung aller Bauteile oder nur der Strahlungsquellen oder nur der Detektoren oder der Strahlungsquellen und Detektoren als voneinander thermisch getrennte Gruppen und eine damit verbundene effiziente Kühlung auf das Niveau der Umgebungstemperatur. Als Effekt erwärmen sich die Bauteile für jede Messung von der Umgebungstemperatur ausgehend und zeigen somit bei jedem Messzyklus eine gleichartige Charakteristik. Ein sich über die Messungen aufbauender Offset aufgrund der Temperatureinflüsse ist somit unterbunden. Die Wärmeabfuhr muss dementsprechend groß sein, um eine Temperaturerhöhung der Bauteile auf einige Kelvin zu begrenzen.

Gleiche Überlegungen wie für die in verschiedenem Abstand d positionierten Detektoren gelten für den die Strahlung der Strahlungsquelle(n) erfassenden Referenzdetektor. Dieser kann in die Stabilisierungsmaßnahmen und Korrekturmöglichkeiten für die Detektoren einbezogen werden.

Die entsprechende erfindungsgemäße **Sensorgestaltung** wurde damit beschrieben. Im Weiteren folgt die mit dem Sensor durchgeführte vorteilhafte **Gestaltung der Messung bzw. Messauswertung.**

Der Messablauf des Sensors sieht vor, für jede Messung eine Dunkelkorrektur durchzuführen, mit der die bei nicht aktivierter Strahlungsquelle(n) auf den bzw. die Detektoren auftreffende Strahlungsmenge und ein von der Signalerfassungsvorrichtung verursachter Offset (Dunkelwert) ermittelt wird.

Ebenfalls kann zur Erfassung der Störungen ohne Minderung des Erfindungsgedankens auch eine Durchleuchtung eines parallelen Strahlverlaufes (Referenzzweig) ohne den Messgegenstand erfolgen.

Im folgenden Schritt wird die ermittelte Störung und / oder der Dunkelwert von der mit aktivierter Strahlungsquelle auf den oder die jeweiligen Detektoren auftreffenden Strahlungsmenge, welche den Offset und / oder die Störung ebenfalls enthält, abgezogen und damit die Störung durch Fremdlicht und Charakteristika der Signalerfassungsvorrichtung deutlich verringert. Die dadurch erhöhte Genauigkeit führt zur Quantifizierung geringerer Konzentrationsänderungen der Zielsubstanz.

Weiterhin sieht der Messablauf zur Erhöhung der Genauigkeit die Wiederholung von Messungen vor, die zeitlich so hintereinander erfolgt, dass keine physiologischen und/oder durch Fremdlicht hervorgerufenen Einflüsse betont werden. Die Wiederholung von Messabläufen bei gleicher Messsituation führt durch Mittelwertbildung vor oder nach der Auswertung zur Verringerung der Einflüsse des Rauschens und anderer Störgrößen, da deren Einflüsse - wenn sie vollständig erfasst werden - sowohl positiv wie negativ auf den Messwert am Detektor einwirken. Die Wiederholung von Messabläufen bei gleicher Messsituation führt ebenfalls zur Verringerung der Einflüsse von zeitlich variablen Störungen. Insbesondere sind die Frequenz der Umgebungslichtquellen (50 Hz bzw. 60 Hz, je nach Netzversorgungsspannungsfrequenz bzw. in der Lichtleistung 100 Hz bzw. 120 Hz) sowie die durch den Herzschlag oder allgemeine, stoffwechselabhängige Regelmechanismen bei Messungen an Haut hervorgerufenen Blutpulsationen, Bewegungsartefakten oder Atembewegungen, bzw. bei Messungen an anderen Messgegenständen deren Frequenzen der sich wiederholenden Vorgänge sowie deren Vielfache hierbei zu beachten. Dabei sind verschiedene Strategien für verschiedene Typen von Störungen vorzusehen:

Wenn die Störung sinusförmig mit konstanter Frequenz ist, ist die Mittelung von zwei Messungen im zeitlichen Abstand von $1/(2 \cdot \text{Störfrequenz})$ vorteilhaft.

Weiterhin bevorzugt ist die Messung über eine oder mehrere vollständige Perioden der Störung, da sich die Einflüsse dann zu einem konstanten Beitrag mitteln, die man durch den Abgleich bei der Auswertung der Messsignale mit einer Referenzmethode zur Konzentrationsbestimmung der gewünschten Zielsubstanz (Vorgehen siehe unten bei der Beschreibung der Auswertung) erfassen und korrigieren kann.

Als weitere Strategie kann die genutzte Messdauer und deren Wiederholungsfrequenz bei Mittelung über sehr lange Zeiten im Verhältnis zur Störungsperiode vorteilhafter Weise nicht mit den Störfrequenzen oder deren Vielfachen übereinstimmen.

Die Zahl der Wiederholungen sollte möglichst groß sein, begrenzt durch die angestrebte Messdauer bis zur Bestimmung der Zielgröße und durch unerwünschte, den Mittelwert der detektierten Strahlungsmenge beeinflussende Veränderungen im Messgegenstand, z. B. bei Haut durch thermische Regulationsprozesse mit Zeitkonstanten im Minutenbereich.

Vorteilhaft kann auch die Lock-In-Technik mit von den Störfrequenzen, und zum Erzielen einer hohen Genauigkeit ihren Vielfachen, verschiedenen Frequenzen genutzt werden, um Störungen zu unterdrücken.

Eine weitere vorteilhafte Lösung zur Reduzierung von optischen Störungen ist die Erfassung der einwirkenden Störgrößen als Störsensorsignale in unmittelbare Nähe des Sensors oder am Sensor, um diese dann gezielt zu korrigieren. Diese Korrektur kann durch Gegenkopplung der erfassten und intensitätskorrigierten Störsensorsignale mit den Messwerten des Sensors erfolgen oder ebenfalls erfindungsgemäß durch Verarbeitung der Störsensorsignale in einer Rechenvorrichtung erfolgen.

Die Messungen werden nach dem Aufbringen auf Messgegenstände mit einem eigenen Regelungssystem vorzugsweise erst nach einer kurzen Pause, d.h. nach der Einschwingzeit des Systems, ausgewertet. Beispielhaft ist hier die Haut zu nennen, da anfangs durch das Aufsetzen des Sensors eine Blutverdrängung in der Haut stattfindet, die sich nach kurzer Zeit zu einem den Einwirkungen angepassten Blutfluss stabilisiert. Während dieser Einschwingzustände stellen sich andere Mittelwerte der detektierten Strahlungsmenge ein und werden vorteilhafter Weise nicht in eine oben beschriebene Mittelung über eine längere Dauer einbezogen.

Die (s.u.) Vorhersage der Quantität einer oder mehrerer Zielsubstanzen bzw. **Auswertung der Messsignale,** d.h. der durch die Detektoren mit unterschiedlichen Abständen d ermittelten Strahlungsmengen, erfolgt entsprechend dem Stand der Technik durch eine Vorhersagefunktion für die in dem jeweiligen Zielvolumen vorhandene Menge an Zielsubstanz mit den für jede Wellenlänge und jeden Abstand d gemessenen (dunkelwertkorrigierten) Strahlungsanteilen. Die Vorhersagefunktion wird durch eine Regressionsanalyse (Chemometrie) entwickelt. Die zur Erstellung, bzw. Kalibration der Vorhersagefunktion genutzten Messungen werden mit dem Sensor an einem Satz von biologischen Geweben bzw. Stoffgemischen vorgenommen, wobei die gesamte Variationsbreite der Gemischzusammensetzung mit den Konzentrationen aller unterschiedlichen Bestandteile abgedeckt wird. Die Konzentration der zu bestimmenden Zielsubstanz wird jeweils durch ein Referenzverfahren ermittelt.

Alternativ kann das zu messende Stoffgemisch durch optische Standards (Phantom) ersetzt werden, wobei unter optischen Standards (Phantom) ein künstlich aus Materialien zusammengemischtes Stoffgemisch verstanden wird, welches ermöglicht, die Vielfalt des eigentlichen Stoffgemischs sowie die geometrischen Abmessungen nachzubilden. Ebenfalls kann der optische Standard (Phantom) eine aktive Vorrichtung darstellen, die die Intensität der Strahlungsquellen erfasst und entsprechend der erwarteten Strahlungsmengen an den Detektorpositionen die geforderte Lichtmenge auf die im Sensor eingebauten Detektoren leitet. Durch die Zugabe der zu ermitelnden Substanz oder durch Referenzmethoden ist deren Konzentration bekannt und kann für die Regressionsverfahren genutzt werden.

Ebenfalls denkbar ist es den Zusammenhang zwischen Sensorsignalen und Konzentration der zu ermitelnden Substanz durch Simulationsrechnungen zu bestimmen. Dies setzt eine genaue Kenntnis der Charakteristik des Sensors und eine exakte Beschreibung der Lichtausbreitung im Stoffgemisch voraus. Hierfür können beispielsweise Monte-Carlo-Simulationen der Lichtausbreitung zur numerischen Lösung der Strahlungstransportgleichung genutzt werden.

Mit einzubeziehen in diese Vorhersagefunktion ist die Datenvorverarbeitung, die in einer erfindungsgemäßen Ausführung darin besteht, die am Detektor gemessenen Rückstreusignale durch Umrechnung in Absorptionsdaten zu transformieren. Eine solche Transformation kann z.B. durch Logarithmieren oder andere mathematische Operationen erfolgen. Aus den durch Logarithmieren näherungsweise in Absorptionsdaten transformierten Messwerten kann durch Regressionsanalyse (Chemometrie) die gesuchte Stoffkonzentration mit weniger Aufwand bestimmt werden. Die Regressionsanalyse (Chemometrie) dient zur Bestimmung der Vorhersagefunktion. Die transformierten Werte werden durch die Vorhersagefunktion in die Stoffkonzentration umgerechnet.

Weitere Vorverarbeitungen bestehen z.B. in der Normierung der Detektormesswerte für jede Wellenlängen auf eine einheitliche Gesamtsumme, wodurch man für die detektierten ortsaufgelösten Rückstreusignale unabhängig von der Strahlungsquellenintensität wird oder in ähnlichen dem Fachmann bekannten Strategien.

Der Aufwand zur Ermittlung der Vorhersagefunktion ist erheblich und kann in dieser oben genannten Form nur für eine kleine Anzahl von Sensoren erfolgen. Durch die Fertigung weisen alle Sensoren eine Varianz auf, z. B. in den Eigenschaften ihrer Bauteile oder deren Anordnung, die zu einem unterschiedlichen Zusammenhang zwischen Konzentration der Zielsubstanz und Sensorsignalen führt, d.h. die Übertragungsfunktion (d.h. Signaltransferfunktion) jedes Sensors muss, wenn die Varianz so groß ist, dass die angestrebte Genauigkeit nicht erreicht werden kann, für die korrekte Funktion des Sensors angepasst werden.

Da hierbei die hohe Empfindlichkeit der Sensoren für geringe Mengen bzw. geringe Änderungen der Zielsubstanz ein wesentliches Kriterium ist, müssen alle Einflüsse auf die Strahlungsmenge an den Detektoren beherrscht werden, was die weiter oben beschriebenen technischen Maßnahmen in Sensorgestaltung und Messablauf gewährleisten. Für eine **Serienfertigung** sollen nicht mühevoll für jeden einzelnen toleranzbehafteten Sensor Messungen an einer Konzentrationsreihe von einer Vielzahl von Stoffgemischen (Messgegenständen) erfolgen, sondern die Kalibrierung wird mit Hilfe von Messungen an wenigen optischen Standards (Phantom) auf den einzelnen Sensor übertragen.

In vorteilhafter Weise gelingt es durch Vergleichsmessungen von kalibrierten Sensoren und nicht-kalibrierten Sensoren an einem oder mehreren optischen Standards (Phantom) eine Rechenvorschrift für die Kalibrierungsübertragung abzuleiten, mit der eine Übertragungsfunktion (Signaltransferfunktion) von den kalibrierten Sensoren auf die nicht-kalibrierten Sensoren übertragen bzw. für die nicht-kalibrierten Sensoren angepasst werden kann. Diese Rechenvorschrift bestimmt die notwendigen Änderungen in der Übertragungsfunktion (Signaltransferfunktion). Die gemessenen Detektormesswerte werden durch die Übertragungsfunktion (Signaltransferfunktion) als Eingangsgrößen für die Vorhersagefunktion der Zielsubstanz transformiert übergeben. Diese Transformation erfolgt derart, dass die übergebenen Werte in einen für alle Sensorsignale gleichartigen Zielwert in einem gemeinsamen Wertebereich überführt werden.

Ebenfalls vorteilhaft ist es, die zu dem individuell kalibrierten Sensor ermittelten Kalibrierungsübertragungsdaten zusammen mit den Messwerten bereitzustellen und in der nachfolgend angewandten Vorhersagefunktion zu berücksichtigen, d.h. bei der Umwandlung von Sensordaten in Konzentrationen der Zielsubstanz.

Der Sensor ist in einer Ausführungsform in Weiterführung des Erfindungsgedankens derart gestaltet, dass eine Rechenvorrichtung als programmierbare Datenverarbeitung integriert ist, welche über den Kanal, der die Messergebnisse nach Anwendung der Übertragungsfunktion (Signaltransferfunktion) durch die programmierte Vorverarbeitung bereitstellt, eine Neueinspielung einer veränderten programmierten Vorverarbeitung erlaubt.

Diese Vorgehensweise zur Übertragung der Kalibrierung gelingt jedoch nur, wenn bestimmte Voraussetzungen erfüllt sind. Der oder die optischen Standards (Phantom), die zur Vergleichsmessung eingesetzt werden, müssen dem realen Messgegenstand in ihren optischen Eigenschaften und geometrischen Abmessungen ähneln, so dass eine ähnliche Strahlungsverteilung zur Messung der Übertragungseigenschaften genutzt werden kann. Bei starker Variationsbreite der optischen Eigenschaften des Messgegenstands müssen mehrere optische Standards (Phantom), die die Variationsbreite abdecken, eingesetzt werden und die Rechenvorschrift für die Kalibrierungsübertragung (Kalibrierungsübertragungsfunktion) ermittelt werden. Bei kleinen Toleranzen bzgl. der Bauteile und Bauteilplatzierung und einer Konstruktion entsprechend der oben beschriebenen Maßnahmen gelingt es eine lineare Kalibrierungsübertragungsfunktion zu realisieren, so dass ein Phantom zur Kalibrierungsübertragung ausreicht. Ist die Kalibrierungsübertragungsfunktion nichtlinear, so kann die Übertragung mit mehreren optischen Standards (Phantom) durchgeführt werden.

Weiterhin sollten für die Kalibrierungsübertragung starke Abhängigkeiten der Übertragungsfunktion (Signaltransferfunktion) der Sensoren oder Vorhersagefunktion von Toleranzen bzw. optischen Eigenschaften des Messgegenstands oder Umgebungsbedingungen konstruktiv und durch das Auswertungsvorgehen vermieden werden. Somit sind die konstruktive Gestaltung des Sensors und die Auswertung direkt mit der Massenfertigung gleichartig genau messender Sensoreinheiten verknüpft.

Durch die Kombination der geschilderten Auswerteverfahren mit einer Auswahl gleichartiger Bauteile und den Maßnahmen zur Sensorgestaltung kann erreicht werden, dass die Varianzen der Sensoreinheiten, d.h. die individuellen Übertragungsfunktionen (Signaltransferfunktion), einen sehr kleinen Beitrag zur Verminderung der Genauigkeit haben, wenn eine Kalibrierungsübertragung genutzt wird und diese linear ist.

Wesentliches Kriterium für die genutzten optischen Standards (Phantom) zur Kalibrierungsübertragung ist eine gleichartige Lichtverteilung, bei ähnlichen geometrischen Abmessungen, wie im Messgegenstand. Dies bezieht sich nicht nur auf gleichartige optische Parameter, sondern auf Aspekte der Lichtverteilung aufgrund mechanischer Eigenschaften, Oberflächeneigenschaften, usw. Eine gleichartige Lichtverteilung wird erreicht durch Nutzung von optischen Standards (Phantom) mit optischen Parametern im Bereich der auftretenden Varianz am Messgegenstand sowie durch Bedingungen an der Oberfläche des optischen Standards (Phantom), die denen des Messgegenstandes derart sind, dass keine unterschiedliche Wirkung auf die Lichtverteilung erfolgt.

Dies äußert sich auch wesentlich dadurch, dass die Wirkung der Strahlungsbarriere auf die Strahlungsverteilung im Messgegenstand und dem bzw. den optischen Standards (Phantom) zur Kalibrierungsübertragung gleichartig ist.

Die Kalibrierungsübertragung für eine Serienfertigung erfolgt also in zwei Schritten. An einem oder mehreren künstlichen Kalibrierungsübertragungshilfsmittel, z.B. einem optischen Standard (Phantom) mit derartigen optischen Parametern, dass die Strahlungsverteilung ähnlich wie am realen Messgegenstand ist, erfolgt mit einem bereits kalibrierten Sensor eine Referenzmessung. Im zweiten Schritt erfolgt durch Signalabgleich (Vergleich) dieser Referenzmessung mit der gleichartig durchgeführten Messung von einem nicht-kalibrierten Sensor an gleichen optischen Standards (Phantom) der Übertrag der Kalibrierung durch Parameteränderungen der Vorhersagefunktion mit der Kalibrierungsübertragungsfunktion.

Im Folgenden wird die Erfindung und Ausgestaltungen der Erfindung beschrieben.

Das Ziel der Erfindung wird durch eine ortsauflösende optische Sensorvorrichtung mit mehreren Strahlungsquellen oder mit einer Quelle mit mehreren Wellenlängenbereichen und mehreren Strahlungsdetektoren oder mit einem Strahlungsdetektor mit mehreren getrennt auslesbaren Untereinheiten zur ortsaufgelösten Erfassung von Zielsubstanzen in stark streuenden Messgegenständen und einer Strahlungsbarriere, die ausgebildet ist Strahlung wenigstens eines Wellenlängenbereiches zu absorbieren und/oder zu reflektieren erreicht. Die Strahlungsquellen sind in einem definierten Abstand zu den Strahlungsdetektoren angeordnet und durch die Strahlungsbarriere von den Strahlungsdetektoren derart getrennt angeordnet, dass die von den Strahlungsquellen erzeugten Strahlungen bevor sie auf die Strahlungsdetektoren fallen, zuerst eine Weglänge durch den Messgegenstand durchlaufen.

In einer Ausgestaltung ragt die Strahlungsbarriere über eine vorgesehene Kontaktfläche mit einem Messgegenstand zwischen 0,1 mm und 1 mm hinaus.

In einer Ausgestaltung umfasst die Strahlungsbarriere ein Bauteile tragendes Substrat und/oder ein die Strahlungsquellen umgebendes Gehäuse und blockiert damit die Strahlungsweiterleitung im Substrat und/oder die Strahlungsweiterleitung durch das Gehäuse.

In einer Ausgestaltung umschließen weitere Strahlungsbarrieren jeden Detektor mit einer umgrenzten Öffnung an der Kontaktfläche zum Messgegenstand.

In einer Ausgestaltung durchläuft die Strahlung von einer der Quellen zu einem in einem bestimmten Abstand positionierten Detektor den Messgegenstand bis zu einer vorbestimmten Tiefe.

In einer Ausgestaltung sind mindestens zwei Strahlungsdetektoren mit unterschiedlichen Abständen zu den Strahlungsquellen angeordnet.

In einer Ausgestaltung weist die Vorrichtung mindestens zwei Strahlungsquellen mit mindestens zwei unterschiedlichen Wellenlängenbereichen auf, die derart gewählt sind, dass mindestens je eine Wellenlänge oder ein Wellenlängenbereich benutzt wird, in dem für jede zu ermittelnde Zielsubstanz die Sensorsignale eindeutige Auswirkungen auf die von dem bzw. den Strahlungsdetektor(en) erfassten Strahlungsmengen zeigen. Zur Erfassung von Störsubstanzen oder anderen Störeinflüssen kann mindestens eine Wellenlänge oder ein Wellenlängenbereich benutzt werden, in dem eine oder mehrere Zielsubstanzen sehr geringe bis keine Auswirkungen auf die Sensorsignale haben, d.h. für die von dem bzw. den Strahlungsdetektor(en) erfassten Strahlungsmengen zeigen die Störsubstanzen jedoch größere Auswirkungen auf die Sensorsignale.

In einer Ausgestaltung der Vorrichtung kann mindestens eine Strahlungsquelle mit mehr als zwei Wellenlängen oder Wellenlängenbereichen derart benutzt werden, dass für jede Störgröße mindestens eine Wellenlänge oder ein Wellenlängenbereich eingestrahlt wird und dass durch die Verwendung von mehr als einer Quelle im Wellenlängenbereich der Zielsubstanz die Empfindlichkeit für diesen Stoff erhöht wird oder Subklassen der Zielsubstanz aufgelöst werden können.

Erfindungsgemäß werden die Signale der Detektoren in verschiedenen Abständen derart verstärkt, dass ähnliche Signalamplituden für alle Detektoren bewirkt werden oder dass die Fläche oder Anzahl der Detektoren für einen bestimmten Abstand jeweils so gewählt wird, dass die Signalamplituden für alle Abstände ähnlich sind.

In einer Ausgestaltung der Vorrichtung kann jeweils nach Einschalten einer Strahlungsquelle eine Art von Signalen mit den Detektoren erfasst werden und nach Ausschalten der Strahlungsquelle eine zweite Art von Signalen mit den Detektoren erfasst werden und durch Subtraktion der beiden Arten von Signalen ein weiter auszuwertendes Ergebnissignal gewonnen werden oder die Signale bei unterschiedlicher Intensität der Strahlungsquelle erfasst und voneinander subtrahiert werden und dadurch ein weiter auszuwertendes Ergebnissignal gewonnen werden.

Ein definierter Teil der Strahlung der Quellen kann in einer Ausgestaltung auf einen Referenzdetektor geleitet werden und mit dem Referenzdetektor kann ein Signal mit eingeschalteter Strahlungsquelle und nachfolgend mit ausgeschalteter Strahlungsquelle erfasst werden, die beiden Signale subtrahiert werden, um ein Referenzsignal zu erhalten und die jeweiligen Ergebnissignale für jede Quelle separat durch dieses so erhaltene Referenzsignal dividiert werden, um ein intensitätsunabhängiges Signal für die weitere Auswertung zu erhalten.

In einer Ausgestaltung werden als Strahlungsquellen gehäuselose Leuchtdioden verwendet, die sehr nahe aneinander platziert werden können, so dass der Messgegenstand von allen Leuchtdioden nahezu gleichartig bestrahlt werden kann.

In einer Ausgestaltung umfasst die Sensorvorrichtung eine Temperatureinstelleinheit. Die Temperatureinstelleinheit kann die Temperatur der Sensorvorrichtung einstellen, insbesondere ist die Temperatureinstelleinheit dazu ausgebildet Strahlungsquellen, beispielsweise Leuchtdioden, oder Detektoren oder beides zu erwärmen und/oder zu kühlen, so dass die Temperatur der Strahlungsquellen auf einen vorgewählten Temperaturwert eingestellt werden können.

In einer Ausgestaltung wird eine breitbandige Lichtquelle als Strahlungsquelle verwendet. In diesem Fall wird als Strahlungsdetektor für die Strahlungsdetektion ein Spektrometer oder ein mit wechselbaren Filtern versehenes Bauteil verwendet.

In einer Ausgestaltung wird die Strahlung der Strahlungsquellen mit Hilfe eines oder mehrerer optischer Elemente, wie z.B. Lichtleiter, Linse, Prisma, Glasstab, Spiegel, Strahlteiler, Fenster, Vergussmasse oder andere optische Elemente auf den Messgegenstand geleitet. Die aus dem Messgegenstand austretende Strahlung kann mit Hilfe eines oder mehrerer anderer optischer Elemente oder denselben optischen Elementen auf die Strahlungsdetektoren geleitet werden.

In einer Ausgestaltung sind die Strahlungsquellen, die Strahlungsdetektoren und alle optischen Elemente mit einer so geringen Toleranz ausgeführt, dass die Übertragung einer Kalibrierung mit ausreichender Genauigkeit erfolgen kann.

Die Genauigkeitsanforderung an die Signale ergibt sich hierbei aus einer Fehlerfortpflanzung zum Fehler des Vorhersagewerts der Zielgröße. Der mathematische Zusammenhang wird über die partielle Ableitung der Vorhersagefunktion nach dem Signal hergestellt:

$$Fehler\ des\ Zielwertes = \sqrt{\sum_{alle\ Signale\ n}\left(\frac{\partial\ Vorhersagefunktion}{\partial\ Signal\ n} \cdot Fehler\ Signal\ n\right)^2}$$

In einer Ausgestaltung erfolgt die Messung aller Signale gleichartig mehrfach wiederholt und eine Mittelung aller in gleicher Weise erfassten Signale erfolgt.

(Auswertung, Kalibrierung, Massenfertigung)

In einer Ausgestaltung wird mit Hilfe einer Sensorvorrichtung an Messgegenständen oder Stoffgemischen mit dem Messgegenstand ähnlichen Eigenschaften hinsichtlich Zielsubstanz sowie Störgrößen eine Messreihe durchgeführt, bei der die Konzentration der Zielsubstanz als Referenzwert gezielt variiert wird oder durch ein anderes Verfahren bei unterschiedlichen Messgegenständen vorbekannt ist und bei der Störungen variiert werden und eine Kalibriervorschrift ermittelt wird, die eine Vorhersage der Konzentration der Zielsubstanz ermöglicht und damit der Sensorvorrichtung als Referenzsensorvorrichtung mit einer Referenzkalibrierung für eine nachfolgende Kalibrierungsübertragung zur Verfügung steht.

In einer Ausgestaltung werden für die Referenzkalibrierung als Signalvorverarbeitungsschritte zuvor der Logarithmus der Ergebnissignale oder der normierten Signale ermittelt und die Referenzwerte mit einer gebrochenen Potenz in die Eingangsgröße für die Vorhersagefunktion umgerechnet.

In einer Ausgestaltung wird die Referenzkalibrierung mit einem Kalibrierungsübertragungsmittel auf weitere bauähnliche Sensorvorrichtungen übertragen, indem Vergleichsmessungen mit Referenzsensorvorrichtung und den bauähnlichen Sensorvorrichtungen vorgenommen werden und die Signalübertragungsfunktion der bauähnlichen Sensorvorrichtung bei zu großer Abweichung angepasst werden.

In einer Ausgestaltung sind das Kalibrierungsübertragungsmittel ein oder mehrere künstliche Messgegenstände, beispielsweise Stoffgemische, die ähnliche optische Eigenschaften hinsichtlich Absorption und Streuung aufweisen und deren Eigenschaften mit einem separaten Verfahren überprüft werden können, so dass eine Veränderung im Laufe der Alterung überprüft werden kann.

In einer Ausgestaltung kann aus den Messungen der Referenzsensorvorrichtung und der bauähnlichen Sensorvorrichtungen an den Kalibrierungsübertragungsmitteln eine Kalibrierungsübertragungsfunktion abgeleitet werden, die die verbliebenen Unterschiede zwischen Referenzsensorvorrichtung und bauähnlichen Sensorvorrichtungen soweit rechnerisch reduziert, dass die Signalhöhen der bauähnlichen Sensorvorrichtungen nach Verrechnung mit der Übertragungsfunktion (Signaltransferfunktion) entsprechend der Kalibrierungsübertragungsfunktion denen der Referenzsensorvorrichtung entsprechen und nachfolgend die Vorhersagefunktion der Referenzsensorvorrichtung zur Vorhersage der Konzentration der Zielsubstanz bei den bauähnlichen Sensorvorrichtungen mit keinem bis sehr geringem Verlust der Genauigkeit angewandt werden kann.

In einer Ausgestaltung können auf die Signale der bauähnlichen Sensorvorrichtungen die gleichen Vorverarbeitungsschritte angewandt werden wie für die Referenzsensorvorrichtung. In diesem Fall sind die Signale in einer mathematisch geordneten Struktur zusammengefasst, so dass diese mit der Übertragungsfunktion (Signaltransferfunktion) elementweise verrechnet und damit in dem Referenzsensor entsprechende Signalhöhen überführt werden können und nachfolgend elementweise mit der (für alle Sensorvorrichtungen gleichen) Vorhersagefunktion zu einer Vorhersage der Konzentration der Zielsubstanz verrechnet werden können.

Spezifische Ausführungsformen

In einer Ausgestaltung ist bzw. sind die in der Haut zu messende Zielsubstanz bzw. Zielsubstanzen Antioxidantien, also Flavonoide oder Karotinoide, insbesondere beta-Karotin, Lykopin, Lutein, Zeaxanthin oder Capsanthin, die in der Epidermis und Dermis gemessen werden.

In einer Ausgestaltung werden Strahlungsquellen verwendet, die im Wellenlängenbereich 380 bis 800 nm liegen und durch schmalbandige Strahlungsquellen, beispielsweise Lichtquellen mit Mittenwellenlängen von 405 nm, 430 nm oder 435 nm, 470 nm, 500 nm, 525 nm und 700 nm realisiert werden. Als Strahlungsdetektoren können beispielsweise Silizium-Photodioden mit einer Kantenlänge von 1 mm bei Mittenabständen zu den Lichtquellen von 1 bis 7 mm, oder auch ähnliche Aufbau-Geometrien zum Einsatz kommen.

In einer Ausgestaltung sind die Strahlungsquellen und Strahlungsdetektoren zur Haut hin durch jeweils ein Fenster abgeschlossen. Zwischen den Strahlungsquellen und Strahlungsdetektoren kann sich eine Strahlungsbarriere befindet, die mit den Fenstern zur Haut hin bündig abschließt oder auch darüber hinausstehen kann.

In einer Ausgestaltung sind die Strahlungsquellen und Strahlungsdetektoren jeweils durch eine Vergussmasse umschlossen. Auch in diesem Fall kann sich zwischen ihnen eine Strahlungsbarriere befinden, die durch Einsägen und Verguss mit einer absorbierenden Masse oder durch ein vormontiertes Bauteil mit ausreichender Strahlungsdämpfung gebildet werden kann. Bevorzugt schließt die Strahlungsbarriere jeweils zur Haut hin bündig ab oder steht darüber hinaus.

In einer Ausgestaltung können verschiedene Abstände zwischen Strahlungsquelle und Strahlungsdetektor getrennt ausgewertet werden und so beispielsweise ein Karotinoidgehalt (Antioxidantienwert) für die Epidermis bei kleinen Abständen im Bereich 0,5 mm bis 4 mm und für die Dermis bei größeren Abständen von 2 mm bis 8 mm ermittelt werden.

In einer Ausgestaltung können die Antioxidantien für verschiedene Unterklassen getrennt ausgewertet werden, indem jeweils eine Strahlungsquelle für jede Unterklasse so gewählt wird, dass deren Mittenwellenlänge der Wellenlänge eines Absorptionsmaximums der Unterklasse von Antioxidantien entspricht.

In einer Ausgestaltung wird die Vorhersage des Antioxidantienwertes in der Sensorvorrichtung berechnet und über eine Kommunikationsschnittstelle an ein Ausgabegerät, Anzeigegerät oder Mobilgerät übertragen.

In einer Ausgestaltung ist die zu messende Zielsubstanz Wasser, das in der Haut in unterschiedlichen Konzentrationen in der Epidermis, Dermis und der Subkutis gemessen werden kann und zeitabhängig als Flüssigkeitseinlagerung bewertet wird bei einer Herzinsuffizienz oder auch für die Bewertung einer ausreichenden Flüssigkeitszufuhr oder der Nierenfunktion.

In einer Ausgestaltung werden Strahlungsquellen im Wellenlängenbereich 900 bis 2100 nm verwendet und durch schmalbandige Strahlungsquellen, beispielsweise Lichtquellen mit Mittenwellenlängen von 975 nm, 1160 nm, 1220 nm, 1320 nm, 1470 nm und 1070 nm realisiert.

Als Strahlungsdetektoren können Indium-Galliumarsenid-Photodioden mit einer Kantenlänge von 1 mm bei Mittenabständen zu den Lichtquellen von 2 bis 12 mm, oder auch ähnliche Aufbau-Geometrien zum Einsatz kommen, um einen Wassergehalt zu ermitteln oder vorherzusagen.

In einer Ausgestaltung sind die Strahlungsquellen und Strahlungsdetektoren jeweils durch ein Fenster, das transparent oder als Band-Pass für den Wellenlängenbereich der Strahlungsquellen ausgeführt sein kann, abgeschlossen. Dazwischen kann eine Strahlungsbarriere angeordnet sein, die zur Haut hin bündig abschließen kann oder auch darüber hinausstehen kann.

In einer Ausgestaltung sind die Strahlungsquellen und Strahlungsdetektoren jeweils durch eine Vergussmasse abgeschlossen. In diesem Fall ist bevorzugt zwischen den Strahlungsquellen und Strahlungsdetektoren eine Strahlungsbarriere angeordnet, die durch Einsägen und Verguss mit einer absorbierenden Masse erfolgt oder durch ein vormontiertes Bauteil mit ausreichender Strahlungsdämpfung, wobei die Strahlungsbarriere jeweils zur Haut hin bündig abschließt oder auch darüber hinausstehen kann oder die Quellen durch ein Prisma oder ähnliches strahlungsübertragendes Element zum Austrittsfenster hin übertragen werden können.

In einer Ausgestaltung werden die Strahlungsquellen von den Detektoren getrennt angeordnet und durch eine Strahlungsbarriere getrennt, wobei die Strahlungsbarriere jeweils zur Haut hin bündig abschließt oder auch darüber hinausstehen kann und die Quellen durch ein Prisma oder ähnliches strahlungsübertragendes Element zum Austrittsfenster hin übertragen werden können.

In einer Ausgestaltung können verschiedene Abstände zwischen Strahlungsquelle und Strahlungsdetektor getrennt ausgewertet werden, so dass ein Wassergehalt für die Dermis bei kleinen Abständen im Bereich 1 mm bis 6 mm und für die Subkutis bei größeren Abständen von 3 mm bis 15 mm ermittelt werden kann.

In einer Ausgestaltung kann der Wassergehalt anhand von wellenlängenabhängigen Unterschieden der Eindringtiefe für die Dermis und die Subkutis getrennt ausgewertet werden, indem Strahlungsquellen mit geringer Wasserabsorption und damit hoher Eindringtiefe für die Subkutis verwendet werden und Strahlungsquellen mit höherer Wasserabsorption und damit geringerer Eindringtiefe für die Dermis verwendet werden.

In einer Ausgestaltung kann der Einfluss des Wassergehalts in der Epidermis als Störgröße aus den Berechnungen des Wassergehalts in der Dermis und der Subkutis eliminiert werden, indem die unterschiedliche Tiefenwichtung bei den verschiedenen Abständen der Strahlungsdetektoren zu den Strahlungsquellen für die Auswertung verwendet wird.

In einer Ausgestaltung sind die zu messenden Zielsubstanzen Hämoglobin und oxygeniertes Hämoglobin sowie der Hämatokrit im Blut in einem extrakorporalen Blutkreislauf während der Dialyse oder der Apherese von Blut oder einer anderen Situation, bei der ein Teil des Blutes sich in einem Schlauchsystem oder einer Küvette als Messstelle befindet.

In einer Ausgestaltung werden Strahlungsquellen im Wellenlängenbereich 380 bis 900 nm verwendet und durch schmalbandige Strahlungsquellen, beispielsweise Lichtquellen mit Mittenwellenlängen $\pm$ Toleranzbereich von 730nm $\pm$ 30 nm, 807,5nm $\pm$ 2,5 nm und 850nm $\pm$ 20 nm realisiert. Als Strahlungsdetektoren können beispielsweise Silizium-Photodioden mit einer Kantenlänge von 1 bis 3 mm bei Mittenabständen zu den Strahlungsquellen von 2 bis 12 mm oder auch ähnliche Aufbau-Geometrien je nach Dimension des Schlauchsystems, bzw. der Küvette, verwendet werden.

In einer Ausgestaltung können die Strahlungsquellen und Strahlungsdetektoren jeweils durch ein Fenster abgeschlossen sein und sich zwischen den Strahlungsquellen und Strahlungsdetektoren eine Strahlungsbarriere befinden, die mit den Fenstern zur Küvette oder dem Schlauch hin abschließt oder auch darüber hinausstehen kann.

In einer Ausgestaltung sind die Strahlungsdetektoren alle von gleicher Art und auf einem Material mit guter Wärmeleitfähigkeit angeordnet, so dass sie eine gleichartige Charakteristik und gleiche Temperatur aufweisen.

In einer Ausgestaltung sind die zu messenden Zielsubstanzen Fett, Wasser und Eiweiß in tierischem Gewebe oder Fleischprodukten, die entweder wie gewachsen oder in einem Verarbeitungsprozess teilweise homogenisiert vorliegen.

In einer Ausgestaltung weist die Sensorvorrichtung Strahlungsquellen im Wellenlängenbereich 900 bis 2500 nm auf, die durch schmalbandige Strahlungsquellen, beispielsweise Lichtquellen mit Mittenwellenlängen von 910 nm, 1200 nm, 1450 nm, 1550 nm, 1680 nm und 1720 nm realisiert sind. Als Strahlungsdetektoren können beispielsweise Indium-Galliumarsenid-Photodioden mit einer Kantenlänge von 1 bis 3 mm bei Mittenabständen zu den Strahlungsquellen von 2 bis 12 mm, oder auch ähnliche Aufbau-Geometrien, zum Einsatz kommen um einen getrennten Fett-, Wasserund Eiweißgehalt zu ermitteln oder vorherzusagen.

In einer Ausgestaltung sind die zu messenden Größen eine Lichtdämpfung in der Epidermis und ein Sonnenschutzfaktor in der Haut durch ein Sonnenschutzmittel.

In einer Ausgestaltung werden Strahlungsquellen im UV, sichtbaren und nahinfraroten Wellenlängenbereich verwendet und können zwischen 280 nm und 1100 nm liegen und beispielsweise durch breitbandige Lichtquellen, wie Xenon-Lichtquellen realisiert werden. Zur Strahlungsdetektion kann als Strahlungsdetektor ein Spektrometer, beispielsweise mit Silizium-Strahlungsdetektoren zum Einsatz kommen.

In einer Ausgestaltung werden Strahlungsquellen im UV, sichtbaren und nahinfraroten Wellenlängenbereich verwendet und können zwischen 280 nm und 1100 nm liegen und beispielsweise durch mindestens eine schmalbandige Lichtquelle, wie Leuchtdioden realisiert werden. Zur Strahlungsdetektion kommt ein Silizium-Strahlungsdetektor zum Einsatz.

In einer Ausgestaltung kann die Strahlung der Strahlungsquellen durch einen oder mehrere Lichtwellenleiter zur Haut übertragen werden. Die optional in verschiedenen Abständen von den Lichtwellenleitern aus der Haut austretende Strahlung kann durch einen oder mehrere Lichtwellenleiter zur Strahlungsdetektion durch das Spektrometer übertragen werden. Die Lichtwellenleiter können einen Durchmesser von 50 bis 600 μm aufweisen. Die Lichtwellenleiter können durch ihre optischen Eigenschaften selbst eine Strahlungsbarriere darstellen oder es kann durch Einbringen von Einbettmitteln oder absorbierenden Hülsen diese Barrierefunktion der Lichtwellenleiter verstärkt werden.

In einer Ausgestaltung sind die verschiedenen Lichtwellenleiter von Strahlungsquellen und Strahlungsdetektoren jeweils einem Abstand zugeordnet. In diesem Fall kann daraus eine Lichtdämpfung durch die Haut ermittelt werden, sowie ein Teil der Abstände eine Ermittlung der Dämpfung alleine durch die Epidermis oder Teile der Epidermis ermöglichen.

In einer Ausgestaltung kann eine wellenlängenabhängige Ermittlung der Dämpfung der Strahlung durch die Epidermis oder Teile der Epidermis vor und nach dem Aufbringen von Sonnenschutzmitteln mittels Quotientenbildung der Dämpfung vor und nach dem Aufbringen der Sonnenschutzmittel die Bestimmung des Sonnenschutzfaktors bzw. eines wellenlängenabhängigen Sonnenschutzfaktors ergeben.

In einer Ausgestaltung ist die zu messende Zielsubstanz Melanin, welches in der Haut in der Epidermis gemessen wird.

In einer Ausgestaltung sind die verwendeten Strahlungsquellen im Wellenlängenbereich 300 bis 800 nm und durch schmalbandige Strahlungsquellen, wie beispielsweise Lichtquellen mit Mittenwellenlängen von 430 nm, 450 nm, 470 nm, 500 nm, 630 nm, 700 nm realisiert. Als Strahlungsdetektoren können beispielsweise Silizium-Photodioden mit einer Kantenlänge von 1 mm bei Mittenabständen zu den Strahlungsquellen von 1 bis 5 mm, oder auch ähnliche Aufbau-Geometrien, zum Einsatz kommen.

In einer Ausgestaltung sind die Strahlungsquellen und Strahlungsdetektoren jeweils durch ein Fenster abgeschlossen. Zwischen den Strahlungsquellen und Strahlungsdetektoren kann eine Strahlungsbarriere angeordnet sein, die mit den Fenstern zur Haut hin abschließt oder auch darüber hinausstehen kann.

In einer Ausgestaltung werden verschiedene Abstände von Strahlungsquellen und Strahlungsdetektoren verwendet, um den Einfluss der Dermis und Subkutis aus der Berechnung des Melaninwertes für die Epidermis herauszurechnen.

In einer Ausgestaltung sind die Strahlungsquellen und Strahlungsdetektoren jeweils durch eine Vergussmasse abgeschlossen. Dazwischen kann eine Strahlungsbarriere angeordnet sein, die durch Einsägen und Verguss mit einer absorbierenden Masse erzeugt werden kann oder durch ein vormontiertes Bauteil mit ausreichender Strahlungsdämpfung erzeugt werden kann, wobei die Strahlungsbarriere jeweils zur Haut hin bündig abschließt oder auch darüber hinausstehen kann.

In einer Ausgestaltung kann aus dem Melaningehalt ein Wert für den Hauttyp abgeleitet werden.

In einer Ausgestaltung ist die zu messende Zielsubstanz Bilirubin, welche in der Haut in der Dermis gemessen wird.

In einer Ausgestaltung weisen die verwendeten Strahlungsquellen einen Wellenlängenbereich von 300 bis 800 nm auf und sind durch schmalbandige Strahlungsquellen, beispielsweise Lichtquellen mit Mittenwellenlängen von 430 nm, 450 nm, 470 nm, 500 nm, 630 nm, 700 nm realisiert. Als Strahlungsdetektoren können beispielsweise Silizium-Photodioden mit einer Kantenlänge von 1 mm bei Mittenabständen zu den Lichtquellen von 1 bis 5 mm, oder auch ähnliche Aufbau-Geometrien zum Einsatz kommen.

In einer Ausgestaltung sind die Strahlungsquellen und Strahlungsdetektoren jeweils durch ein Fenster abgeschlossen. Dazwischen kann eine Strahlungsbarriere angeordnet sein, die mit den Fenstern zur Haut hin abschließt oder auch darüber hinausstehen kann.

In einer Ausgestaltung können die verschiedenen Abstände von Strahlungsquellen und Strahlungsdetektoren verwendet werden, um den Einfluss der Epidermis und Subkutis aus der Berechnung des Bilirubingehaltes für die Dermis herauszurechnen.

In einer Ausgestaltung sind die Strahlungsquellen und Strahlungsdetektoren jeweils durch eine Vergussmasse abgeschlossen. Dazwischen kann eine Strahlungsbarriere angeordnet sein, die durch Einsägen und Verguss mit einer absorbierenden Masse erzeugt werden kann oder durch ein vormontiertes Bauteil mit ausreichender Strahlungsdämpfung gebildet werden kann, wobei die Strahlungsbarriere jeweils zur Haut hin bündig abschließt oder auch darüber hinausstehen kann.

Beschreibung der Zeichnungen

Fig. 1    Exemplarische Lösung eines Sensormoduls mit Chip-LED

Fig. 2    Exemplarischer Aufbau einer verzahnten Anordnung eines Sensors

Fig. 3    Lösungen für eine optische Kopplung des Sensors mit einem Messgegenstand (Probe)

Fig. 4    Exemplarische Lösung der Temperatur-Stabilisierung eines Bauteile tragenden Substrates

Fig. 5      Ausführungsbeispiel Antioxidantiensensor

Fig. 6      Ausführungsbeispiel Wassergehaltssensor

Fig. 7      Ausführungsbeispiel für die Sensorplatine des Hämatokrit- und Sauerstoffsättigungs-Sensors

Fig. 8      Ausführungsbeispiel für den Platinenträger des Hämatokrit- und Sauerstoffsättigungs-Sensors

Fig. 9      Ausführungsbeispiel Fleischzusammensetzungssensor

Fig. 10      Ausführungsbeispiel Sonnenschutzfaktor-Sensor

Fig. 11      Ausführungsbeispiel Bilirubinsensor bzw. Melaninsensor

Die in den folgenden Zeichnungen dargestellten Ausführungsbeispiele sind konkrete Ausführungen der oben im Text beschriebenen allgemeinen technischen Lösungen.

Antioxidantien-Sensor

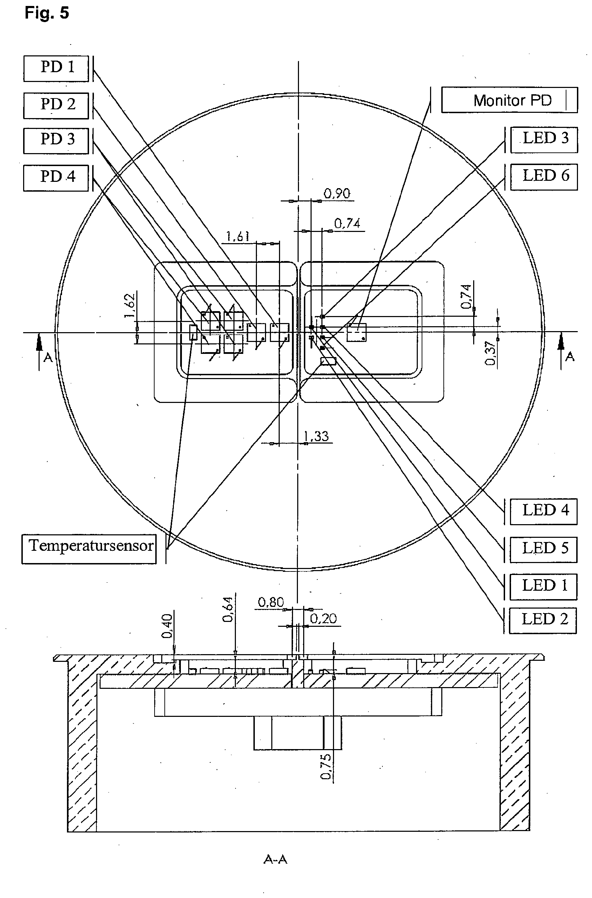

Ein Ausführungsbeispiel für einen Sensor zur Erfassung von antioxidativ wirkenden Stoffen in der Haut, insbesondere Beta-Karotin und Lykopin, wird in **Fig. 5** gezeigt. Die Hauptstörgrößen bei der Erfassung sind Hämoglobin und Melanin. Der Sensor umfasst sechs Strahlungsquellen jeweils als Chip-LED mit 300 $\mu$m Seitenlänge, die mit den Mittenwellenlängen 405 nm, 435 nm, 470 nm, 500 nm, 525 nm, 700 nm sowie sechs Chip-Photodioden aus Silizium als Detektoren mit 1.000 $\mu$m Seitenlänge mit den Mittenabständen LED1 zu PD1 = 2,23 mm, LED1 zu PD2 = 3,84 mm, LED1 zu PD3 = 5,45 mm, LED1 zu PD4 = 7,06 mm sowie einer Monitor-Photodiode (Monitoring PD), die im Abstand von 2,6 mm von LED1 aber in entgegengesetzter Richtung zu den anderen Photodioden angeordnet ist und identisch zu den oben genannten Photodioden ist. Die als PD3 und PD4 bezeichneten Photodioden sind nebeneinander mit dem Abstand 1,62 mm zueinander angeordnet und elektrisch vor dem Verstärker parallel geschaltet. LED2 ist angeordnet auf dem Sensorboard (oder auch Sensorplatine) in einer zur Achse LED1-PD4 rechtwinkligen Achse mit Abstand 0,74 mm zu LED1. LED3 bis LED6 sind angeordnet auf einer parallel zur Achse LED1-LED2 befindlichen Achse, die 0,74 mm weiter von den PD entfernt ist. Zwischen den LED3 bis LED6 besteht jeweils ein Abstand von 0,74 mm.

Die Wellenlängen der LED können um einige Nanometer abweichen, ohne die Funktion des Sensors zu beeinträchtigen.

Die LEDs sind auf einem Lichtquellenboard und die Photodioden auf einem Detektorboard zusammen mit jeweils einem Temperatursensor auf der gleichen Oberfläche montiert, so dass alle Bauteile auf der gleichen Seite platziert sind. Beide Boards sind gemeinsam auf einen Träger aus einer schwarz eloxierten Aluminiumlegierung montiert, welcher jeweils eine Aussparung von je mit 6 x 7.8 mm$^2$ enthält für den Lichtdurchlass über dem Lichtquellenboard und dem Detektorboard, welche mit einem transparenten Fenster aus Glas flüssigkeitsdicht verschlossen sind. Zwischen den Fenstern und den beiden Boards ist eine Strahlungsbarriere im Träger ausgeführt.

Die Größe der Aussparungen kann vorteilhaft verkleinert werden oder auch anders gestaltet werden entsprechend den in der obigen Beschreibung dargestellten Prinzipien oder dem Stand der Technik, ohne dass der Sensor seine Genauigkeit verändert, sofern das Signal der Detektoren nicht im Rauschen untergeht.

Beide Boards zusammen bilden das Sensorboard, welches wiederum verbunden ist mit einem Signalerfassungsboard mit für jeden Sensor getrennter Verstärkung und Analog-zu-Digital-Wandlung der Signale sowie einem weiteren Board mit einem Mikroprozessor zur Steuerung der Signalerfassung. Dieses Mikroprozessorboard umfasst ebenfalls eine Kommunikationsschnittstelle, die als drahtlose und kabelgebundene Schnittstelle nach dem Stand der Technik ausgeführt sein kann. Die Kommunikation und Steuerung der Energieversorgung kann ebenfalls auf einem getrennten Board ausgeführt und elektrisch mit den übrigen Boards verbunden sein.

Ein anderer Aufbau der Funktionseinheiten ist denkbar, ohne das Wesen der Erfindung zu verändern. Insbesondere die Platzierung der Analog-zu-Digital-Wandlung kann mit den Detektoren auf ein Board und auch die Strahlungsquellen und Detektoren können auf einem Board platziert sein.

Im Mikrocontroller erfasst eine Firmware-Routine die digitalen Signale und logarithmiert diese. Andere Datenvorverarbeitungen wie oben ausgeführt sind ebenfalls vorteilhaft. Mit Hilfe einer Kalibrierfunktion wird nachfolgend eine Vorhersage für die Konzentration der Antioxidantien in der Haut über die Kommunikationsschnittstelle herausgegeben. Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix (LEDi x PDj) vor, die durch elementweise Multiplikation auf eine korrigierte Übertragungsfunktion (Signaltransferfunktion) mit einem Standardsignalbereich transformiert werden.

Der zweite Teil der Kalibrierfunktion, die Vorhersagefunktion, ermittelt die Vorhersage der Konzentration der Zielsubstanz. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu der Stoffkonzentration verrechnet. Vorteilhaft erfolgt diese Berechnung der Vorhersagefunktion mit vier verschiedenen Koeffizientensätzen, wobei der gewichtete Mittelwert der so ermittelten Konzentrationen der Zielsubstanz die Ausgabe des Sensors ist.

Der Messort für den Sensor ist im Betrieb generell die Haut, wobei Areale ohne Haare und mit kleiner Krümmung bevorzugt sind. Besonders geeignete Messorte sind die beiden Handballen unter dem Daumen bzw. dem kleinen Finger, da dort ausreichend dicke Epidermis vorhanden ist, um eine Trennung von Epidermis und den tieferliegenden Schichten mit nur geringem Antioxidantiengehalt zu gewährleisten.

Eine alternative Ausführungsform beinhaltet eine geänderte geometrische Anordnung der LED und PD auf einem gemeinsamen Board derart, dass Schlitze die LED und Photodioden jeweils voneinander und von der restlichen Platine thermisch entkoppeln.

In Weiterführung können die Strahlungsquellen und Detektoren als integriertes Bauteil (Modul) mit gleicher optischer Geometrie aufgebaut und auf das Sensorboard aufgebracht werden. Somit ersetzt das Modul das Lichtquellenboard sowie das Detektorboard. Das Modul wird nach Kontaktieren durch Bonden mit einer transparenten Vergussmasse aus Epoxidharz (z.B. Hysol OS 4000 der Fa. Henkel) mechanisch geschützt. Zwischen den Lichtquellen und Detektoren wird durch Einsägen durch die Vergussmasse bis zum Board und anschließenden Verguss des Sägespalts mit stark absorbierender Vergussmasse nachträglich eine Strahlungsbarriere eingefügt. In gleicher Weise werden Barrieren zwischen den Photodioden eingebracht, wobei allerdings nicht bis zum Board eingesägt wird. Das Modul kann auch nur die Strahlungsquelle oder nur die Detektoren mit dem vorgegebenen Abstand beinhalten und wird in gleicher Weise wie beim vorangegangenen Ausführungsbeispiel anstelle des Lichtquellenboards bzw. des Detektorboards eingesetzt und wie oben zu einem vollständigen Sensor zusammengebaut.

Auf die gleiche Weise wird in Weiterführung ein Sensormodul aufgebaut, wobei alle oben beschriebenen Boards ebenfalls in den vergossenen Block integriert werden.

Wassergehaltssensor

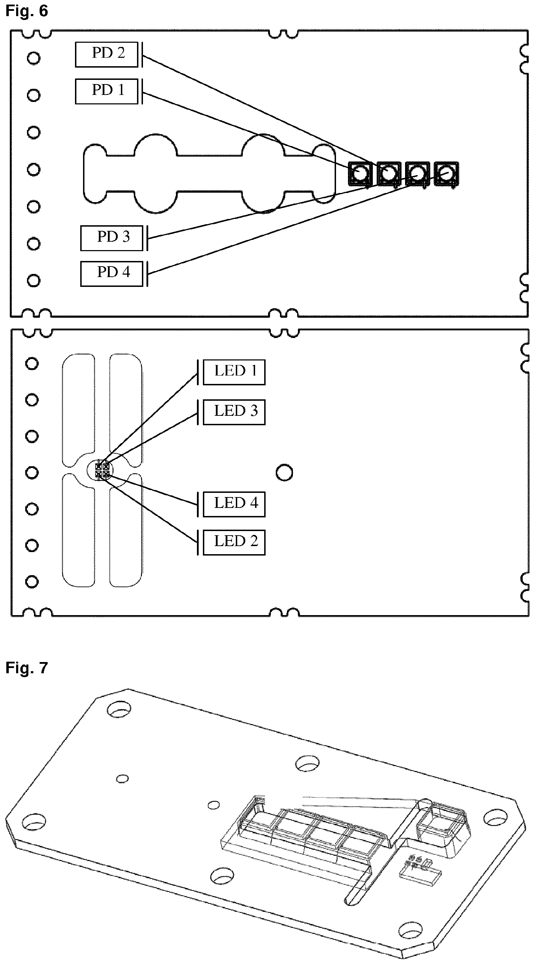

In **Fig. 6** wird ein Ausführungsbeispiel für einen Sensor zur Erfassung des Wassergehalts (Konzentration von Wasser) in der Haut, insbesondere in der Dermis und Subkutis gezeigt. Die Hauptstörgröße stellt die Epidermis mit Schweißbedeckung und einem unterschiedlichen Wassergehalt, der Melaningehalt der Haut und unterschiedliche Lichtstreuung je nach Zustand der Haut dar. Der Sensor umfasst vier Strahlungsquellen jeweils als Chip-LED mit 300 $\mu$m Seitenlänge, die mit den Mittenwellenlängen $\pm$ Toleranzbereich 975 $\pm$ 5 nm, 1070 $\pm$ 15 nm, 1160 $\pm$ 15 nm, 1320 $\pm$ 20 nm, sowie vier Chip-Photodioden aus Indium-Galliumarsenid (InGaAs) als Detektoren mit 1.000 $\mu$m Seitenlänge.

Ebenfalls denkbar ist ein Austausch der LED-Wellenlänge 1160 nm mit einer Wellenlänge zwischen 1150 bis 1220 nm.

Alle LED strahlen in ein Umlenkprisma mit den Maßen 14 mm x 2,5 x 2,5 mm$^2$, welches als Lichtmischer fungiert und eine homogene und gleiche Verteilung des Lichts jeder LED bewirkt. Das Licht wird vom Umlenkprisma durch ein Fenster mit dem Durchmesser 1,5 bis 4 mm gestrahlt, welches auf der Haut aufliegt. Das Umlenkprisma ist vorteilhaft bis auf die Ein- und Austrittsfläche mit einer absorbierenden Beschichtung überzogen, welche als Strahlungsbarriere ein Übersprechen zu den Strahlungsdetektoren verhindert. Die Strahlungsdetektoren sind auf einer Achse auf dem gleichen Board mit einem Mittenabstand von 2 mm zueinander und einem Abstand von 3 mm der Mitte von PD1 zur Mitte des Austrittsfensters angeordnet. Die PD sind zur Haut hin mit einem Gehäuse abgeschlossen, in das über jeder PD ein Fenster mit Durchmesser 1 bis 3 mm flüssigkeitsdicht eingebracht ist, welches eine hohe Transmission im Spektralbereich der LED aufweist und eine geringe Transmission im sichtbaren Spektralbereich und somit als Tageslichtfilter wirkt.

Die hier genannten Geometrien für die Austrittsfenster und das Umlenkprisma können variiert werden ohne das Wesen der Erfindung zu verändern.

Die LED werden auf einem hohen Temperaturniveau gehalten, welches nahe der maximal erreichbaren Betriebstemperatur, die im Dauerbetrieb ermittelt wurde, liegt. Dies wird mit einem Temperatursensor überwacht. Andere Lösungen gemäß den Ausführungen weiter oben sind ebenfalls vorteilhaft einsetzbar.

Die Strahlungsquellen sind auf einem LED-Board angeordnet, welches elektrisch mit dem Sensorboard verbunden ist und mit diesem gleiche Abmaße aufweist. Vorteilhafterweise wird das LED-Board mit einem Abstandshalter mit dem Sensorboard mechanisch verbunden.

Das Sensorboard hat die Maße 15 x 40 mm$^2$ und trägt die Funktionen der Signalerfassung mit für jeden Sensor getrennter Verstärkung und Analog-zu-Digital-Wandlung der Signale sowie einen Mikroprozessor zur Steuerung der Signalerfassung. Über eine elektrische Verbindung ist ein Kommunikations- und Energieversorgungsboard verbunden, welches eine Kommunikationsschnittstelle, die als drahtlose und kabelgebundene Schnittstelle nach dem Stand der Technik ausgeführt sein kann, umfasst.

Ein anderer Aufbau der Funktionseinheiten sowie andere Außenmaße sind denkbar, ohne das Wesen der Erfindung zu verändern.

Im Mikrocontroller erfasst eine Firmware-Routine die digitalen Signale und logarithmiert diese. Andere Datenvorverarbeitungen wie oben ausgeführt sind ebenfalls vorteilhaft. Mit Hilfe einer Kalibrierfunktion wird nachfolgend eine Vorhersage für den Wassergehalt in der Haut über die Kommunikationsschnittstelle herausgegeben. Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix (LEDi x PDj) vor, die durch elementweise Multiplikation auf eine korrigierte Übertragungsfunktion (Signaltransferfunktion) die Messwertmatrix auf einen Standardsignalbereich transformiert.

Der zweite Teil der Kalibrierfunktion, die Vorhersagefunktion, ist eine Rechenvorschrift zur Ermittlung der Konzentration der Zielsubstanz. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu der Stoffkonzentration verrechnet.

Der Messort für den Sensor ist im Betrieb generell die Haut, wobei für den Zweck der Überwachung bei der chronischen Herzinsuffizienz besonders Messorte am Bein, z.B. im Bereich des Knöchels, in Betracht kommen. Für eine generelle Überwachung des Wasserhaushalts bzw. Wassergehalts kommen auch Messorte am Arm und der Hand in Betracht.

Hämatokrit / Sauerstoffsättigung

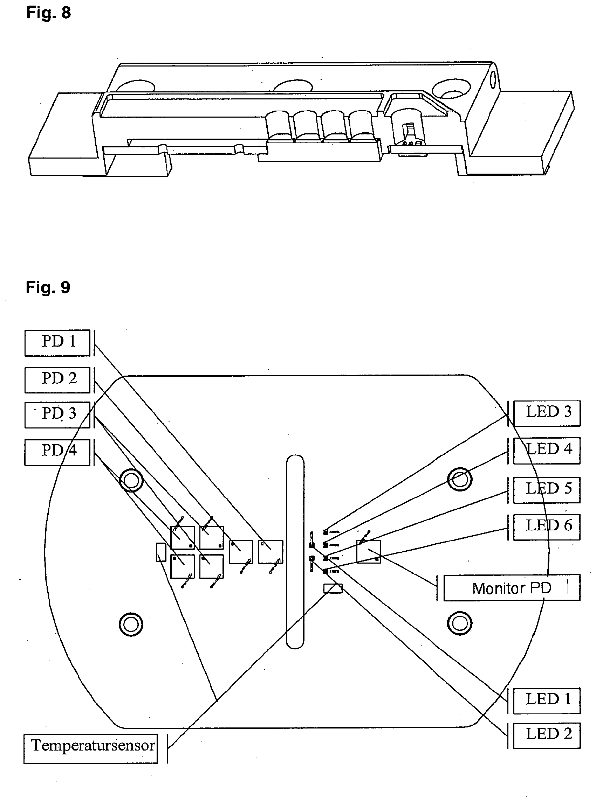

Ein Ausführungsbeispiel für einen Sensor zur Erfassung des Hämatokrit und der Sauerstoffsättigung von Blut, z.B. während Operationen mit Herz-Lungen-Maschinen wird in **Fig. 7** und **Fig. 8** dargestellt. Der Messort ist eine blutdurchströmte Küvette oder ein Schlauch, welche in **Fig. 8** oberhalb der runden Öffnungen platziert werden und als toleranzbehaftete Bauteile bzw. durch verschiedene Ausführungsformen potentielle Störgrößen darstellen. Weiterhin stören Pumpenstöße die Messung der optischen Eigenschaften des Blutes und Blutbestandteile wie Bilirubin und Lipide. Aber auch die Blutzellen selbst weisen eine hohe individuelle Variabilität in Größe und Hämoglobingehalt auf, die störend auf eine Messung wirken.

Der Sensor umfasst vier Strahlungsquellen jeweils als Chip-LED mit 300 $\mu$m Seitenlänge, die mit den Mitten-wellenlängen $\pm$ Toleranzbereich 730 $\pm$ 30 nm (zwei gleiche LED diagonal zueinander angeordnet verbaut), 807,5 $\pm$ 2,5 nm, 850 $\pm$ 20 nm sowie vier Chip-Photodioden aus Silizium als Detektoren mit 2.600 $\mu$m Seitenlänge auf einem gemeinsamen Sensorboard, in welches ein Wärmeleitträger integriert ist, der die Photodioden durch ein thermisch sehr gut leitendes Material, beispielsweise Kupfer oder ein anderes thermisch sehr gut leitendes Material miteinander verbindet und auf einem Temperaturniveau oberhalb der Raumtemperatur bzw. maximalen Temperatur des Messgegenstandes (z.B. 38 °C) gehalten wird. Auf dem Wärmeleitträger im Sensorboard befindet sich eine Monitor-Photodiode, die im Abstand von 5 mm von LED1 rechtwinklig zur Achse LED-PD1-4 angeordnet ist und identisch zu den oben genannten Photodioden ist, die über einen diffusen Reflektor aus Spektralon einen stets gleichen Teil der abgestrahlten Lichtmenge der LED zugeführt bekommt.

Die Abstände der LED zueinander betragen 0,7 mm, wobei die 730 nm-LED zweifach diagonal an den Ecken der LED-Anordnung zueinander bestückt ist. Die Mitte der PD 1 ist 5 mm zum Mittelpunkt der Vier-LED-Anordnung positioniert, die weiteren PD weisen untereinander einen Abstand von 3,2 mm auf und sind in einer Linie angeordnet.

In einem Abstand von 0,7 mm neben der LED3 und LED4 ist ein Temperatursensor montiert und auf der anderen Seite des Wärmeleitträger als Draht symmetrisch mittig unter allen PD ein zweiter Temperatursensor. Unter dem ersten Temperatursensor und den LED ist ein Heizwiderstand (R6 in Fig. 4) mit einstellbarem Strom zum Heizen des Boards gegenüberliegend zu den direkt darüber befindlichen Bauteilen angeordnet und derart elektrisch angesteuert, dass eine Temperaturregelung auf die vorab definierte, maximale Umgebungstemperatur realisiert wird. Zur Wärmeisolierung bzw. Verringerung der Wärmeleitung ist das Board um die beheizten Bauteile herum mit Schlitzen versehen.

Das Sensorboard ist auf einen Träger aus schwarzem PEEK (ähnlicher Ausdehnungskoeffizient wie das Platinensubstratmaterial FR4) montiert, in welchem eine Wandung ausgebildet ist, die in einen Schlitz zwischen LED1 bis LED4 und PD1 bis PD4 als Strahlungsbarriere ragt. Im Träger ist symmetrisch zu LED1 bis LED4 eine zylindrische Bohrung (mit Gewinde zur Verringerung von Störstrahlung bzw. Reflexionen in der Bohrung) mit Durchmesser M4 x 0,5 mm ausgeführt und über jeder Photodiode PD1 bis PD4 ist symmetrisch eine Bohrung (mit Gewinde zur Verringerung von Störstrahlung bzw. Reflexionen in der Bohrung) mit Durchmesser M3 x 0,35 mm ausgeführt. Die Bohrungen über den Photodioden sind mit einem gemeinsamen Glasfenster der Dicke 0,4 mm flüssigkeitsdicht abgeschlossen und die gemeinsame Bohrung über den LED sind mit einem Glasfenster der Dicke 0,4 mm flüssigkeitsdicht abgeschlossen, wobei aus dem Träger heraus die Strahlungsbarriere soweit übersteht, dass die beiden Fenster unterbrochen werden und sich an die Barriere bündig anlegen.

Das Sensorboard ist verbunden mit einem Signalerfassungsboard mit für jeden Sensor getrennter Verstärkung und Analog-zu-Digital-Wandlung der Signale sowie mit einem Mikroprozessor zur Steuerung der Signalerfassung. Dieses Board umfasst ebenfalls eine Kommunikationsschnittstelle, die als drahtlose und kabelgebundene Schnittstelle nach dem Stand der Technik ausgeführt sein kann.

Die Kommunikation und Steuerung der Energieversorgung kann ebenfalls auf einem getrennten Board ausgeführt und elektrisch mit den übrigen Boards verbunden sein.

Ein anderer Aufbau der Funktionseinheiten ist denkbar, ohne das Wesen der Erfindung zu verändern. Insbesondere die Platzierung der Analog-zu-Digital-Wandlung kann mit den Detektoren auf ein Board und auch die Strahlungsquellen und Detektoren können auf einem Board platziert sein.

Im Mikrocontroller erfasst eine Firmware-Routine die digitalen Signale und logarithmiert diese. Andere Datenvorverarbeitungen wie oben ausgeführt sind ebenfalls vorteilhaft. Mit Hilfe einer Kalibrierfunktion wird nachfolgend eine Vorhersage für den Hämatokrit und für den Sauerstoffgehalt des Blutes über die Kommunikationsschnittstelle herausgegeben. Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix (LEDi x PDj) vor, die durch elementweise Multiplikation auf eine korrigierte Übertragungsfunktion (Signaltransferfunktion) mit einem Standardsignalbereich transformiert werden.

Der zweite Teil der Kalibrierfunktion, die Vorhersagefunktion, ermittelt die Vorhersage der Konzentration der Zielsubstanz. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu der Stoffkonzentration verrechnet.

Der Messort für den Sensor ist generell an einem blutgefüllten Behältnis, welches unterschiedliche Abmessungen aufweisen kann und an die der Sensor durch eine streuende, lichtdichte Behältnisaufnahme angepasst wird.

Fleischzusammensetzung

Ein Ausführungsbeispiel für einen Sensor zur Erfassung der Zusammensetzung von Fleisch und Fettgewebe tierischen Ursprungs ("Fleischstücke"), bzw. von zerkleinerten und / oder vermischten Verarbeitungsprodukten hiervon, insbesondere Fett-, Eiweiß- und Wassergehalt, wird in **Fig. 9** beschrieben. Der Sensor umfasst sechs Strahlungsquellen jeweils als Chip-LED mit 300 μm Seitenlänge, die mit den Mittenwellenlängen 910 nm, 1200 nm, 1450 nm, 1550 nm, 1680 nm und 1720 nm mit je ± 20 nm Toleranz sowie sechs Chip-Photodioden aus Indium-Galliumarsenid (InGaAs) als Strahlungsdetektoren mit 1.000 μm Seitenlänge mit den Mittenabständen LED1 zu PD1 = 2,23 mm, LED1 zu PD2 = 3,84 mm, LED1 zu PD3 = 5,45 mm, LED1 zu PD4 = 7,06 mm sowie einer Monitor-Photodiode (Monitoring PD), die im Abstand von 2,6 mm von LED1 aber in entgegengesetzter Richtung zu den anderen Photodioden angeordnet ist und identisch zu den oben genannten Photodioden ist. Die als PD3 und PD4 bezeichneten Photodioden sind nebeneinander mit dem Abstand 1,62 mm zueinander angeordnet und elektrisch vor dem Verstärker parallel geschaltet. LED2 ist angeordnet auf dem Sensorboard (oder auch Sensorplatine) in einer zur Achse LED1-PD4 rechtwinkligen Achse mit Abstand 0,74 mm zu LED1. LED3 bis LED6 sind angeordnet auf einer parallel zur Achse LED1-LED2 befindlichen Achse, die 0,74 mm weiter von den PD entfernt ist. Zwischen den LED3 bis LED6 besteht jeweils ein Abstand von 0,74 mm.

Die LEDs sind auf einem Lichtquellenboard und die Photodioden auf einem Detektorboard zusammen mit jeweils einem Temperatursensor auf der gleichen Oberfläche montiert, so dass alle Bauteile auf der gleichen Seite platziert sind. Beide Boards sind gemeinsam auf einen Träger aus einer schwarz eloxierten Aluminiumlegierung montiert, welcher jeweils eine Aussparung von je mit 6 x 7.8 mm² enthält für den Lichtdurchlass über dem Lichtquellenboard und dem Detektorboard, welche mit einem transparenten Fenster aus Glas flüssigkeitsdicht verschlossen sind. Zwischen den Fenstern und den beiden Boards ist eine Strahlungsbarriere im Träger ausgeführt.

Die Größe der Aussparungen kann vorteilhaft verkleinert werden oder auch anders gestaltet werden entsprechend den in der obigen Beschreibung dargestellten Prinzipien oder dem Stand der Technik, ohne dass der Sensor seine Genauigkeit verändert, sofern das Signal der Detektoren nicht im Rauschen untergeht.

Beide Boards zusammen bilden das Sensorboard, welches wiederum verbunden ist mit einem Signalerfassungsboard mit für jeden Sensor getrennter Verstärkung und Analog-zu-Digital-Wandlung der Signale sowie einem weiteren Board mit einem Mikroprozessor zur Steuerung der Signalerfassung. Dieses Mikroprozessorboard umfasst ebenfalls eine Kommunikationsschnittstelle, die als drahtlose und kabelgebundene Schnittstelle nach dem Stand der Technik ausgeführt sein kann. Die Kommunikation und Steuerung der Energieversorgung kann ebenfalls auf einem getrennten Board ausgeführt und elektrisch mit den übrigen Boards verbunden sein.

Ein anderer Aufbau der Funktionseinheiten ist denkbar, ohne das Wesen der Erfindung zu verändern. Insbesondere die Platzierung der Analog-zu-Digital-Wandlung kann mit den Detektoren auf ein Board und auch die Strahlungsquellen und Detektoren können auf einem Board platziert sein.

Im Mikrocontroller erfasst eine Firmware-Routine die digitalen Signale und logarithmiert diese. Andere Datenvorverarbeitungen wie oben ausgeführt sind ebenfalls vorteilhaft. Mit Hilfe einer Kalibrierfunktion wird nachfolgend eine Vorhersage für die Konzentration von Wasser, Fett und Eiweiß in Fleischstücken über die Kommunikationsschnittstelle herausgegeben. Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix ($LEDi \times PDj$) vor, die durch elementweise Multiplikation auf eine korrigierte Übertragungsfunktion (Signaltransferfunktion) mit einem Standardsignalbereich transformiert werden.

Der zweite Teil der Kalibrierfunktion, die Vorhersagefunktion, ermittelt die Vorhersage der Konzentration der Zielsubstanz. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu den Stoffkonzentrationen verrechnet.

Der Messort für den Sensor ist im Betrieb generell die Oberfläche von Fleisch- oder Fettgewebe, wobei angeschnittene Gewebeflächen ohne Fasziesbedeckung und mit kleiner Krümmung bevorzugt sind oder von Verarbeitungsprodukten hiervon.

Sonnenschutzfaktorenbestimmung

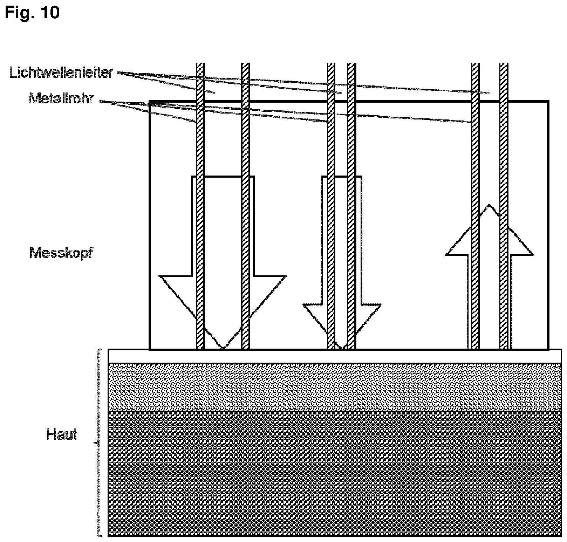

Ein Ausführungsbeispiel für einen Sensor zur Erfassung der Lichtdämpfung der obersten Hautschichten in der Epidermis zur Ermittlung des Lichtschutzes der Haut sowie von Sonnenschutzmitteln ist in **Fig. 10** dargestellt. Der Sensor umfasst eine breitbandige Xenon-Lichtquelle mit Lichtanteilen im UVA, UVB und sichtbaren sowie nahinfraroten Spektralbereich, welche unterbrechbar durch einen ersten Shutter in einen ersten Beleuchtungslichtleiter transmittiert und auf der gegenüberliegenden Seite der Lampe über einen zweiten Shutter Licht in einen zweiten Beleuchtungslichtleiter koppelt. Ein Detektionslichtleiter ist an den Eingangsspalt eines Spektrometers für das UVA, UVB, den sichtbaren und nahinfraroten Spektralbereich gekoppelt, bzw. der Lichtleiter stellt den Eingangsspalt durch seine geometrischen Abmessungen dar. Das von der Strahlungsquelle bzw. Detektor entfernte Faserende der zwei oder mehr Beleuchtungslichtleiter und des mindestens einen Detektionslichtleiters sind zu einem optischen Messkopf zusammengeführt, der mit den Lichtleiterstirnflächen im Betrieb direkt auf die zu messende Stelle der Haut aufgesetzt wird. Der Lichtleiter für die Detektion ist für die zu untersuchenden Spektralbereiche durchlässig, die Lichtleiter für die Beleuchtung können nur teilweise durchlässig sein, wobei die Verluste durch eine stärkere Strahlungsquelle ausgeglichen werden. Die Lichtleiter weisen alle einen Durchmesser im Bereich von 50 bis 600 μm auf, welcher an den jeweils untersuchten Spektralbereich derart angepasst ist, dass die kleinen Durchmesser bei kleiner optischer Eindringtiefe in die Haut, wie z.B. im Ultravioletten, und größere Durchmesser bei großen Durchmesser bei großer optischer Eindringtiefe in die Haut, wie z.B. im roten und nachinfraroten Spektralbereich, genutzt werden. Die Lichtleiter sind in einer Linie angeordnet, wobei der Detektionslichtleiter so angeordnet ist, dass je nach Lichtleiterdurchmesser unterschiedliche Detektionsflächen und durch die Anordnung unterschiedliche Abstände d1 und d2 des Detektionslichtleiters zum ersten bzw. zweiten Beleuchtungslichtleiter entstehen. Die Totalreflexion im jeweiligen Lichtleiter stellt eine erste Strahlungsbarriere zwischen den Lichtleitern dar, der Mantel des Lichtleiters aus Kunststoff oder Metall stellt eine zweite Strahlungsbarriere dar, das Einbettmittel zum Präparieren der Lichtleiteranordnung stellt eine dritte Strahlungsbarriere dar und ein zusätzliches Metallrohr um jede einzelne Faser stellt eine vierte Strahlungsbarriere dar, auf die optional auch verzichtet werden kann.

Für eine Messung werden zuerst alle Shutter geschlossen und ein Dunkelspektrum mit dem Spektrometer gemessen, dann wird Shutter 1 geöffnet und ein Spektrum für den Abstand d1 gemessen, nachfolgend wird Shutter 1 geschlossen, Shutter 2 geöffnet und ein Spektrum für den Abstand d2 gemessen. Die Spektren bei d1 und d2 werden jeweils durch Subtraktion des Dunkelspektrums korrigiert und der Auswertung zugeführt. Vorteilhaft wird die Integrationszeit für die Spektrometermessung an die jeweilige Lichtmenge für die Abstände d1 und d2 angepasst und jeweils ein Dunkelspektrum für die mindestens zwei unterschiedlichen Integrationszeiten getrennt erfasst und den mit dieser Integrationszeit durchgeführten Messungen richtig zugeordnet jeweils subtrahiert.

Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix (Wellenlänge_i x Detektion_j) vor, die durch elementweise Multiplikation auf eine korrigierte Übertragungsfunktion (Signaltransferfunktion) mit einem Standardsignalbereich transformiert werden.

Der zweite Teil der Kalibrierfunktion, die Vorhersagefunktion, ermittelt die Vorhersage eine Lichtdämpfung der Haut. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu der Lichtdämpfung verrechnet.

Bei einer Messung an der Haut wird mit der Kalibrierfunktion für die gemessene Lichtdämpfung eine Vorhersage für die intrinsische spektrale Dämpfung der Haut selbst ermittelt. Wird nachfolgend dieses Hautareal mit einem Sonnenschutzmittel eingecremt, so kann durch Verhältnisbildung der spektralen Lichtdämpfung vor und nach Eincremen der spektrale Sonnenschutzfaktor ermittelt werden.

Der Einfluss durch eine Wartezeit zwischen den beiden Messungen, durch Abwaschen oder durch Reiben mit Kleidung oder durch eine andere Manipulation kann der Einfluss auf den Sonnenschutz bestimmt werden.

Eine alternative Messanordnung zur obigen Messanordnung wird durch einen Beleuchtungslichtleiter und zwei Detektionslichtleiter im Abstand d1 und d2, die entweder an zwei Spektrometer angeschlossen sind, oder an ein Mehrkanalspektrometer zur getrennten Erfassung der beiden Spektren für d1 und d2, erstellt. Der Messvorgang kann damit für die Abstände d1 und d2 zeitgleich erfolgen. Die weitere Messung und Auswertung erfolgt analog zu der obigen Ausführungsform.

Als weitere Alternative werden anstelle der drei einzelnen Lichtleiter ein zentraler einzelner Lichtleiter und zwei konzentrische Ringe von Lichtleitern mit dem Abstand d1 und d2 um den zentralen Lichtleiter ausgeführt. Die beiden Ringe werden jeweils entweder zur Lichtquelle mit zwei Ausgängen geführt oder zu den beiden Spektrometern oder zu dem Multikanalspektrometer mit zwei Eingängen. Die weitere Messung und Auswertung erfolgt analog zu der obigen Ausführungsform.

Bilirubin und Melanin

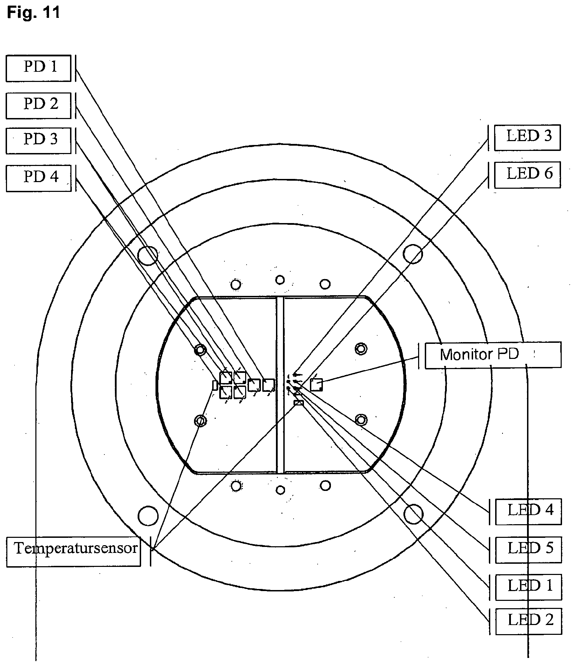

Ein Ausführungsbeispiel für einen Sensor zur Erfassung von Bilirubin in der Haut ist in **Fig. 11** dargestellt. Die Hauptstörgrößen bei der Erfassung sind Hämoglobin bzw. Blut sowie Melanin. Damit ist der Sensor auch zur Bestimmung des Melaningehaltes in der Haut einsetzbar. Der Sensor umfasst sechs Strahlungsquellen jeweils als Chip-LED mit 300 $\mu$m Seitenlänge, die mit den Mittenwellenlängen 430 nm, 450 nm, 470 nm, 500 nm, 630 nm, 700 nm sowie vier Chip-Photodioden aus Silizium als Strahlungsdetektoren mit 1.000 $\mu$m Seitenlänge mit den Mittenabständen LED1 zu PD1 = 2,23 mm, LED1 zu PD2 = 3,84 mm, LED1 zu PD3 = 5,45 mm, LED1 zu PD4 = 7,06 mm sowie einer Monitor-Photodiode, die im Abstand von 2,6 mm von LED1 aber in entgegengesetzter Richtung angeordnet ist und identisch zu den oben genannten Photodioden ist. LED2 ist angeordnet auf dem Sensorboard in einer zur Achse LED1-PD4 rechtwinkligen Achse mit Abstand 0,74 mm. LED3 bis LED6 sind angeordnet auf eine parallel zur Achse LED1-LED2 befindlichen Achse, die 0,74 mm weiter von den PD entfernt ist. Zwischen den LED3 bis LED6 besteht jeweils ein Abstand von 0,74 mm.

Die Wahl der Wellenlängen kann geändert werden, wobei für Bilirubin im blauen Wellenlängenbereich (400-520 nm) mindestens zwei Wellenlängen oder Wellenlängenbereiche ohne erhöhte Blutabsorption gewählt werden und für die Melaninkorrektur, bzw. -messung eine Wellenlänge im blauen Wellenlängenbereich ausreichend ist (weitere hinzuzunehmen ist vorteilhaft), dafür aber mindestens eine Wellenlänge im roten Spektralbereich (600-780 nm) ohne erhöhte Blut- oder Wasserabsorption vorzusehen ist.

Die LEDs sind auf einem Lichtquellenboard und die Photodioden auf einem Detektorboard zusammen mit jeweils einem Temperatursensor auf der gleichen Oberfläche montiert, so dass alle Bauteile auf der gleichen Seite platziert sind. Beide Boards sind gemeinsam auf einen Träger aus einer schwarz eloxierten Aluminiumlegierung montiert, welcher jeweils eine Aussparung von je mit 6 x 7.8 mm$^2$ enthält für den Lichtdurchlass über dem Lichtquellenboard und dem Detektorboard, welche mit einem transparenten Fenster aus Glas flüssigkeitsdicht verschlossen sind. Zwischen den Fenstern und den beiden Boards ist eine Strahlungsbarriere im Träger ausgeführt.

Die Größe der Aussparungen kann vorteilhaft verkleinert werden oder auch anders gestaltet werden entsprechend den in der obigen Beschreibung dargestellten Prinzipien oder dem Stand der Technik, ohne dass der Sensor seine Genauigkeit verändert, sofern das Signal der Detektoren nicht im Rauschen untergeht.

Beide Boards zusammen bilden das Sensorboard, welches wiederum verbunden ist mit einem Signalerfassungsboard mit für jeden Sensor getrennter Verstärkung und Analog-zu-Digital-Wandlung der Signale sowie einem weitern Board mit einem Mikroprozessor zur Steuerung der Signalerfassung. Dieses Mikroprozessor-board umfasst ebenfalls eine Kommunikationsschnittstelle, die als drahtlose und kabelgebundene Schnittstelle nach dem Stand der Technik ausgeführt sein kann. Die Kommunikation und Steuerung der Energieversorgung kann ebenfalls auf einem getrennten Board ausgeführt und elektrisch mit den übrigen Boards verbunden sein.

Ein anderer Aufbau der Funktionseinheiten ist denkbar, ohne das Wesen der Erfindung zu verändern. Insbesondere die Platzierung der Analog-zu-Digital-Wandlung kann mit den Detektoren auf ein Board und auch die Strahlungsquellen und Detektoren können auf einem Board platziert sein.

Im Mikrocontroller erfasst eine Firmware-Routine die digitalen Signale und logarithmiert diese. Andere Datenvorverarbeitungen wie oben ausgeführt sind ebenfalls vorteilhaft. Mit Hilfe einer ersten Kalibrierfunktion wird eine Vorhersage für den Bilirubinwert und mit einer zweiten Kalibrierfunktion, die in einem weiteren Sensor oder im gleichen Sensor implementiert sein kann, wird für den Melaninwert in der Haut die Konzentration bzw. die korrigierte Konzentration über die Kommunikationsschnittstelle herausgegeben. Die Kalibrierfunktion besteht aus zwei Teilen, der Übertragungsfunktion (Signaltransferfunktion) und der Vorhersagefunktion. Der erste Teil, die Übertragungsfunktion (Signaltransferfunktion), ist für den jeweiligen Sensor gültig und wird angepasst durch eine Kalibrierungsübertragungsfunktion, die für den individuellen Sensor die Toleranzen beinhaltet und aus einer Messung mit einem kalibrierten (Referenz-)Sensor und dem individuellen Sensor aus einer Vergleichsmessung an Standards abgeleitet wird. Die unkorrigierte Übertragungsfunktion (Signaltransferfunktion) für den Sensortyp und die Kalibrierungsübertragungsfunktion liegen jeweils als Matrix (LEDi x PDj) vor, die durch elementweise Multiplikation auf eine korrigierte Übertragungsfunktion (Signaltransferfunktion) mit einem Standardsignalbereich transformiert werden.

Der zweite Teil der Kalibrierfunktion, die Vorhersagefunktion, ermittelt die Vorhersage der Konzentration der Zielsubstanz und eliminiert dabei den Einfluss der Störsubstanz oder andere Störeinflüsse, die in den Messungen vorhanden waren. Dieser zweite Teil umfasst die Vorverarbeitung (log) der aus der Übertragungsfunktion (Signaltransferfunktion) erhaltenen Werte und deren Multiplikation mit den Koeffizienten aus der Regressionsanalyse (Chemometrie), die summiert werden und mit einem Offset addiert und über mathematische Operationen wie beispielsweise Wurzelziehen und Quadrieren zu der Stoffkonzentration verrechnet.

Der Messort für den Sensor ist im Betrieb generell die Haut, wobei Areale ohne Haare und mit kleiner Krümmung bevorzugt sind.

**Patentansprüche**

1. Ortsauflösende optische Sensorvorrichtung mit

   - mehreren Strahlungsquellen (LED1-LED6) oder mit einer Quelle mit mehreren Wellenlängenbereichen und
   - mehreren Strahlungsdetektoren (PD1-PD4) oder mit einem Strahlungsdetektor mit mehreren getrennt auslesbaren Untereinheiten zur Bestimmung der Mengen von Zielsubstanzen in stark streuenden Messgegenständen,
   - einer Strahlungsbarriere, die ausgebildet ist Strahlung wenigstens eines Wellenlängenbereiches zu absorbieren und/oder zu reflektieren, wobei die Strahlungsquellen in verschiedenen vorbestimmten Abständen zu den Strahlungsdetektoren angeordnet sind und durch die Strahlungsbarriere von den Strahlungsdetektoren derart getrennt angeordnet sind, dass die von den Strahlungsquellen erzeugten Strahlungen bevor sie auf die Strahlungsdetektoren fallen, zuerst eine Weglänge durch den Messgegenstand durchlaufen, und
   - einer Verstärkereinheit, die ausgebildet ist Signale der Strahlungsdetektoren in den verschiedenen vorbestimmten Abständen derart zu verstärken, dass ähnliche Signalamplituden für alle Strahlungsdetektoren bewirkt werden oder mit einer Fläche der Strahlungsdetektoren, wobei die Fläche der Strahlungsdetektoren für einen vorbestimmten Abstand jeweils so gewählt ist, dass die Signalamplituden für alle vorbestimmten Abstände ähnlich sind, wobei eine der eingestrahlten Wellenlängen oder ein Wellenlängenbereich geeignet ist, auf die Sensorsignale der zu ermittelnde Zielsubstanz, d.h. die von dem bzw. den Detektoren erfassten Strahlungsmengen, eine Auswirkung aufzuweisen, und
   wobei zur Erfassung von Störsubstanzen oder anderen Störeinflüssen mindestens eine der eingestrahlten Wellenlängen oder einer der eingestrahlten Wellenlängenbereiche geeignet ist, auf die Sensorsignale der zu ermittelnde Zielsubstanz, d.h. die von dem bzw. den Detektoren erfassten Strahlungsmengen, keine Auswirkungen aufzuweisen,

wobei die Zielsubstanz eine Substanz oder mehrere Substanzen aus der Gruppe der Antioxidantien, Wasser, Hämoglobin, oxidiertes Hämoglobin, Hämatokrit, Fett, Eiweiß, Melanin, Sonnenschutzmittel und Bilirubin ist, wobei immer mindestens ein Paar von Auswirkung-zeigender und keine Auswirkung zeigender Wellenlänge der Strahlung in gleichem Abstand d einstrahlbar sind.

2. Sensorvorrichtung gemäß Anspruch 1, wobei die Strahlungsbarriere ein Bauteile tragendes Substrat und/oder ein die Strahlungsquelle (LED1-LED6) umgebendes Gehäuse umfasst und damit die Strahlungsweiterleitung im Substrat und/oder die Strahlungsweiterleitung durch das Gehäuse blockiert.

3. Sensorvorrichtung gemäß Anspruch 1 oder 2, wobei wenigstens eine Strahlungsquelle (LED1-LED6) derart ausgebildet und/oder angeordnet ist, dass die Strahlung von der Strahlungsquelle den Messgegenstand bis zu einer vorbestimmten Tiefe durchläuft.

4. Sensorvorrichtung gemäß wenigstens einem der Ansprüche 1 bis 3, wobei die Abstände von mindestens zwei der Strahlungsdetektoren (PD1-PD4) zu den Strahlungsquellen (LED1-LED6) so gewählt sind, dass die Weglänge der Strahlung im Zielvolumen für die zu erfassende wenigstens eine Zielsubstanz maximiert ist und sich die Messvolumen für die unterschiedlichen Strahlungsdetektoren zumindest teilweise überlappen.

5. Sensorvorrichtung gemäß wenigstens einem der Ansprüche 1 bis 4, wobei die Strahlungsquellen (LED1-LED6) und Strahlungsdetektoren (PD1-PD4) derart angeordnet sind, dass eine Tiefenwichtung durch die unterschiedlichen Abstände derart gewählt ist, dass der Einfluss störender oberflächennaher Volumina oder der Einfluss tieferer Volumina als das Zielvolumen durch eine Verrechnung der Signale bei unterschiedlichen Abständen reduziert ist.

6. Sensorvorrichtung gemäß wenigstens einem der Ansprüche 1 bis 5, wobei mindestens eine der Strahlungsquellen (LED1-LED6) derart ausgebildet ist, dass für jede Störgröße mindestens eine Wellenlänge oder ein Wellenlängenbereich eingestrahlt werden kann und dass durch die Verwendung von mehr als einer Wellenlänge oder Wellenlängenbereich in der Sensorvorrichtung die Empfindlichkeit für diesen Stoff erhöht ist und/oder Subklassen der Zielsubstanz aufgelöst sind.

7. Sensorvorrichtung gemäß wenigstens einem der Ansprüche 1 bis 6, wobei die Sensorvorrichtung eine Temperatureinstellungseinheit aufweist, die ausgebildet ist die Strahlungsquellen (LED1-LED6) zu erwärmen und/oder zu kühlen, so dass die Temperatur der Strahlungsquellen auf einen vorbestimmten Temperaturwert einstellbar ist.

8. Vorrichtung nach Anspruch 1 bis 7, wobei die Sensorvorrichtung ausgebildet ist, an Messgegenständen oder an Stoffgemischen mit gegenüber dem Messgegenstand ähnlichen Eigenschaften hinsichtlich Zielsubstanz sowie Störgrößen eine Messreihe durchzuführen, bei der die Konzentration der Zielsubstanz als Referenzwert gezielt variiert werden kann oder durch ein anderes Verfahren bei unterschiedlichen Messgegenständen vorbekannt ist und bei der Störungen variiert werden können und eine Kalibriervorschrift ermittelt werden kann, die eine Vorhersage der Konzentration der Zielsubstanz ermöglicht und damit die Sensorvorrichtung als Referenzsensorvorrichtung mit einer Referenzkalibrierung für eine nachfolgende Kalibrierungsübertragung zur Verfügung steht.

9. Sensorvorrichtung gemäß Anspruch 8, wobei die Sensorvorrichtung ein Kalibrierungsübertragungsmittel aufweist und/oder mit einem Kalibrierungsübertragungsmittel verbunden werden kann, das ausgebildet ist die Referenzkalibrierung auf weitere bauähnliche Sensorvorrichtungen zu übertragen, indem Vergleichsmessungen mit der Referenzsensorvorrichtung und den bauähnlichen Sensorvorrichtungen vorgenommen werden und eine Signalübertragungsfunktion der bauähnlichen Sensorvorrichtung bei zu großer Abweichung angepasst werden kann.

10. Sensorvorrichtung gemäß wenigstens einem der Ansprüche 1 bis 9, wobei die Sensorvorrichtung im Betrieb ausgebildet ist Zielsubstanzen in einer Haut eines Nutzers zu messen und wobei die in der Haut zu messende Zielsubstanz Antioxidantien, also Flavonoide oder Karotinoide sind, insbesondere beta-Karotin, Lykopin, Lutein, Zeaxanthin oder Capsanthin, die in der Epidermis und Dermis zu messen sind, oder die zu messenden Zielsubstanzen Fett, Wasser und Eiweiß in tierischem Gewebe oder Fleischprodukten sind, die entweder wie gewachsen oder nach einem Verarbeitungsprozess vorliegen oder die zu messende Zielsubstanz Melanin ist, welches in der Haut in der Epidermis zu messen sind, oder die zu messende Zielsubstanz Bilirubin ist, welche in der Haut in der Dermis zu messen sind, oder die in der Haut zu messende Zielsubstanz Wasser ist, das in der Haut in unterschiedlichen Konzentrationen in der Epidermis, Dermis und der Subkutis zu messen ist und zeitabhängig als Flüssigkeitseinlagerung bei einer Herzinsuffizienz, für eine Bewertung einer ausreichenden Flüssigkeitszufuhr oder für eine Bewertung einer Nierenfunktion zu bewerten ist.

11. Sensorvorrichtung gemäß wenigstens einem der Ansprüche 1 bis 9, wobei die Sensorvorrichtung im Betrieb ausgebildet ist Zielsubstanzen Hämoglobin und/oder oxygeniertes Hämoglobin zu messen und/oder den Hämatokrit im Blut zu bestimmen in einem extrakorporalen Blutkreislauf während der Dialyse oder der Apherese von Blut oder einer anderen Situation, bei der ein Teil des Blutes sich in einem Schlauchsystem oder einer Küvette als Messstelle befindet.

12. Sensorvorrichtung gemäß Anspruch 10 oder 11, wobei die Strahlungsquellen (LED1-LED6) und Strahlungsdetektoren (PD1-PD4) zum Messgegenstand hin durch jeweils ein Fenster abgeschlossen werden und sich zwischen ihnen eine Strahlungsbarriere befindet, die mit den Fenstern zum Messgegenstand hin bündig abschließt oder auch darüber hinausstehen kann.

13. Sensorvorrichtung gemäß einem der Ansprüche 10 bis 12, wobei die Sensorvorrichtung ausgebildet ist Zielsubstanzen für verschiedene Unterklassen getrennt auszuwerten, indem jeweils eine Strahlungsquelle (LED1-LED6) für jede Unterklasse so gewählt ist, dass deren Mittenwellenlänge einer Wellenlänge eines Absorptionsmaximums der Unterklasse von Zielsubstanzen entspricht.

14. Sensorvorrichtung gemäß wenigstens einem der Ansprüche 10 bis 13, wobei die Sensorvorrichtung ausgebildet ist einen Vorhersagewert der Zielsubstanz aus der Messung der Zielsubstanz zu ermitteln und wobei die Sensorvorrichtung eine Kommunikationsschnittstelle aufweist, die mit einem Ausgabegerät verbunden ist oder verbindbar ist und die Kommunikationsschnittstelle ausgebildet ist den Vorhersagewert der Zielsubstanz an das Ausgabegerät zu übertragen.

15. Sensorvorrichtung gemäß wenigstens einem der Ansprüche 10 bis 14, wobei die Sensorvorrichtung ausgebildet ist einen Wassergehalt anhand von wellenlängenabhängigen Unterschieden einer Eindringtiefe für die Dermis und die Subkutis getrennt auszuwerten, indem Strahlungsquellen (LED1-LED6) mit geringer Wasserabsorption und damit hoher Eindringtiefe für die Subkutis vorgesehen sind und Strahlungsquellen mit höherer Wasserabsorption und damit geringerer Eindringtiefe für die Dermis vorgesehen sind.

16. Verfahren zur ortsauflösenden Erfassung von Zielsubstanzen mit folgenden Verfahrensschritten:

   - Einstrahlen von Licht aus mehreren Strahlungsquellen (LED1-LED6) oder aus einer Quelle mit mehreren Wellenlängenbereichen und
   - Detektion von Messwerten in mehreren Strahlungsdetektoren (PD1-PD4) oder in einem Strahlungsdetektor mit mehreren getrennt auslesbaren Untereinheiten zur Bestimmung der Mengen von Zielsubstanzen in stark streuenden Messgegenständen,
   - Anordnen einer Strahlungsbarriere, wobei die Strahlungsbarriere ausgebildet ist Strahlung wenigstens eines Wellenlängenbereiches zu absorbieren und/oder zu reflektieren, wobei die Anordnung der Strahlungsquellen in verschiedenen vorbestimmten Abständen zu den Strahlungsdetektoren erfolgt und wobei die Strahlungsquellen durch die Strahlungsbarriere von den Strahlungsdetektoren derart getrennt angeordnet sind, dass die von den Strahlungsquellen erzeugten Strahlungen bevor sie auf die Strahlungsdetektoren fallen, zuerst eine Weglänge durch den Messgegenstand durchlaufen,
   - Verstärkung der Signale der Strahlungsdetektoren, dass ähnliche Signalamplituden für alle Strahlungsdetektoren erreicht werden, oder
   Auswahl der Fläche der Strahlungsdetektoren, dass ähnliche Signalamplituden für alle Strahlungsdetektoren erreicht werden,
   wobei eine der Wellenlängen oder ein Wellenlängenbereich, die auf die Sensorsignale der zu ermittelnde Zielsubstanz, d.h. die von dem bzw. den Detektoren erfassten Strahlungsmengen, eine Auswirkungen aufweist, und
   wobei eine der Wellenlängen oder ein Wellenlängenbereich zur Erfassung von Störsubstanzen oder anderen Störeinflüssen, die auf die Sensorsignale der zu ermittelnde Zielsubstanz, d.h. die von dem bzw. den Detektoren erfassten Strahlungsmengen, keine Auswirkungen aufweisen,
   wobei die Zielsubstanz eine Substanz oder mehrere Substanzen aus der Gruppe der Antioxidantien, Wasser, Hämoglobin, oxidiertes Hämoglobin, Hämatokrit, Fett, Eiweiß, Melanin, Sonnenschutzmittel und Bilirubin ist,
   wobei die Einstrahlung immer mindestens eines Paares von Auswirkung-zeigender und keine Auswirkung zeigender Wellenlänge der Strahlung in gleichem Abstand d erfolgt.

**Claims**

1.  A high-resolution optical sensor device, having

    - multiple radiation sources (LED1-LED6) or a source with multiple wavelength ranges and
    - multiple radiation detectors (PD1-PD4) or a radiation detector having multiple separately readable sub-units to determine the amounts of target substances in highly scattering measurement objects,
    - a radiation barrier which is designed to absorb and/or reflect radiation of at least one wavelength range, wherein the radiation sources are arranged at different predetermined distances from the radiation detectors and are arranged separated from the radiation detectors by the radiation barrier in such a way that the radiations generated by the radiation sources first travel a path length through the measurement before impinging on the radiation detectors, and
    - an amplifier unit which is designed to amplify signals from the radiation detectors at the various predetermined distances in such a way that similar signal amplitudes are generated for all radiation detectors or with an area of the radiation detectors, wherein the area of the radiation detectors is respectively selected for a predetermined distance so that the signal amplitudes are similar for all predetermined distances, wherein one of the irradiated wavelengths or a wavelength range is suitable for having an effect on the sensor signals of the target substance to be determined, namely the amounts of radiation detected by the detector(s), and

    wherein at least one of the irradiated wavelengths or one of the irradiated wavelength ranges is suitable for having no effect on the sensor signals of the target substance to be determined, namely the amounts of radiation detected by the detector(s), wherein the target substance is one or more substances from the group of antioxidants, water, hemoglobin, oxidized hemoglobin, hematocrit, fat, protein, melanin, sunscreen and bilirubin, wherein at least one pair of effect-showing and no-effect-showing wavelengths of radiation can always be irradiated at the same distance d.

2.  The sensor device according to claim 1, wherein the radiation barrier comprises a substrate carrying components and/or a housing surrounding the radiation source (LED1-LED6) and thus blocks the radiation transmission in the substrate and/or the radiation transmission through the housing.

3.  The sensor device according to claim 1 or 2, wherein at least one radiation source (LED1-LED6) is designed and/or arranged in such a way that the radiation from the radiation source penetrates through the measurement object to a predetermined depth.

4.  The sensor device according to at least any one of claims 1 to 3, wherein the distances from at least two of the radiation detectors (PD1-PD4) to the radiation sources (LED1-LED6) are selected so that the path length of the radiation in the target volume is maximized for the at least one target substance to be detected and the measurement volumes for the different radiation detectors at least partially overlap.

5.  The sensor device according to at least any one of claims 1 to 4, wherein the radiation sources (LED1-LED6) and radiation detectors (PD1-PD4) are arranged in such a way that a depth weighting is selected by the different distances in such a way that the influence of disturbing volumes near the surface or the influence of volumes deeper than the target volume is reduced by processing the signals at different distances.

6.  The sensor device according to at least any one of claims 1 to 5, wherein at least one of the radiation sources (LED1-LED6) is designed in such a way that at least one wavelength or a wavelength range can be irradiated for each disturbance variable and that by using more than one wavelength or wavelength range in the sensor device the sensitivity for this substance is increased and/or sub-classes of the target substance are resolved.

7.  The sensor device according to at least any one of claims 1 to 6, wherein the sensor device has a temperature setting unit which is designed to heat and/or cool the radiation sources (LED1-LED6) so that the temperature of the radiation sources can be set to a predetermined temperature value.

8.  The device according to claims 1 to 7, wherein the sensor device is designed to carry out a series of measurements on measurement objects or on mixtures of substances with properties similar to the measurement object with regard to the target substance and disturbance variables, in which the concentration of the target substance can be varied in a targeted manner as a reference value or is already known for different measurement objects by using another method and in which disturbances can be varied and a calibration rule can be determined which enables a prediction of the concentration of the target substance and thus the sensor device is available as a reference sensor device with a reference calibration for subsequent calibration transfer.

9. The sensor device according to claim 8, wherein the sensor device has a calibration transfer means and/or can be connected to a calibration transfer means, which is designed to transfer the reference calibration to further structurally similar sensor devices by carrying out comparison measurements with the reference sensor device and the structurally similar sensor devices and a signal transfer function of the structurally similar sensor device can be adapted if the deviation is too large.

10. The sensor device according to at least any one of claims 1 to 9, wherein the sensor device is designed, during operation, to measure target substances in a user's skin and wherein the target substance to be measured in the skin are antioxidants, namely flavonoids or carotenoids, in particular beta-carotene, lycopene, lutein, zeaxanthin or capsanthin, which are to be measured in the epidermis and dermis, or the target substances to be measured are fat, water and protein in animal tissue or meat products, which are either provided in a grown state or after a processing process, or the target substance to be measured is melanin, which is to be measured in the skin in the epidermis, or the target substance to be measured is bilirubin, which is to be measured in the skin in the dermis, or the target substance to be measured in the skin is water, which is present in the skin in different concentrations in the epidermis, dermis and subcutis and is to be assessed time-dependently as fluid retention in case of heart failure, for an assessment of adequate fluid intake or for an assessment of kidney function.

11. The sensor device according to at least any one of claims 1 to 9, wherein the sensor device is designed, in operation, to measure target substances hemoglobin and/or oxygenated hemoglobin and/or to determine the hematocrit in the blood in an extra-corporeal blood circulation during dialysis or apheresis of blood or another situation in which part of the blood is present in a tubing system or a cuvette as a measuring station.

12. The sensor device according to claim 10 or 11, wherein the radiation sources (LED1-LED6) and radiation detectors (PD1-PD4) are each closed off from the measurement object by a window and with a radiation barrier located therebetween, which is flush with the windows towards the measurement object or can also protrude beyond them.

13. The sensor device according to any one of claims 10 to 12, wherein the sensor device is designed to evaluate target substances separately for different sub-classes by selecting a respective radiation source (LED1-LED6) for each subclass so that its center wavelength corresponds to a wavelength of an absorption maximum of the subclass of target substances.

14. The sensor device according to at least any one of claims 10 to 13, wherein the sensor device is designed to determine a prediction value of the target substance from the measurement of the target substance and wherein the sensor device has a communication interface which is connected or can be connected to an output device and wherein the communication interface is designed to transmit the prediction value of the target substance to the output device.

15. The sensor device according to at least any one of claims 10 to 14, wherein the sensor device is designed to separately evaluate a water content based on wavelength-dependent differences in a penetration depth for the dermis and the subcutis, by providing radiation sources (LED1-LED6) with low water absorption and thus high penetration depth for the subcutis and radiation sources with higher water absorption and thus lower penetration depth for the dermis.

16. A method for high-resolution detection of target substances, with the following steps:

   - irradiating light from multiple radiation sources (LED1-LED6) or from a source with multiple wavelength ranges and
   - detecting measurement values in multiple radiation detectors (PD1-PD4) or in a radiation detector having multiple separately readable sub-units to determine the amounts of target substances in highly scattering measurement objects,
   - arranging a radiation barrier,
   wherein the radiation barrier is designed to absorb and/or reflect radiation of at least one wavelength range, wherein the radiation sources are arranged at different predetermined distances from the radiation detectors and wherein the radiation sources are arranged separated from the radiation detectors by the radiation barrier in such a way that the radiations generated by the radiation sources first travel a path length through the measurement object before impinging on the radiation detectors,

- amplifying the signals from the radiation detectors so that similar signal amplitudes are achieved for all radiation detectors, or

selecting the area of the radiation detectors so that similar signal amplitudes are achieved for all radiation detectors, wherein one of the wavelengths or a wavelength range that has an effect on the sensor signals of the target substance to be determined, namely the amounts of radiation detected by the detector(s), and wherein one of the wavelengths or a wavelength range for detecting disturbing substances or other disturbing influences, that have no effect on the sensor signals of the target substance to be determined, namely the amounts of radiation detected by the detector(s),

wherein the target substance is made of one or more substances from the group of antioxidants, water, hemoglobin, oxidized hemoglobin, hematocrit, fat, protein, melanin, sunscreen and bilirubin,

wherein the irradiation with at least one pair of effective and non effective wavelengths of the radiation always takes place at the same distance d.

**Revendications**

1. Dispositif de détection optique à résolution spatiale comprenant

plusieurs sources de rayonnement (LED1-LED6) ou une source ayant plusieurs plages de longueurs d'onde et plusieurs détecteurs de rayonnement (PD1-PD4) ou un détecteur de rayonnement ayant plusieurs sous-unités lisibles séparément pour déterminer les quantités de substances cibles dans des objets de mesure hautement diffusants,

une barrière anti-rayonnement conçue pour absorber et/ou réfléchir un rayonnement d'au moins une plage de longueurs d'onde, dans lequel les sources de rayonnement sont disposées à différentes distances prédéterminées des détecteurs de rayonnement et sont disposées séparées des détecteurs de rayonnement par la barrière anti-rayonnement de sorte que les rayonnements générés par les sources de rayonnement, avant de tomber sur les détecteurs de rayonnement, parcourent d'abord une longueur de trajet à travers l'objet de mesure, et

une unité d'amplification conçue pour amplifier les signaux provenant des détecteurs de rayonnement à diverses distances prédéterminées de sorte que des amplitudes de signal similaires soient provoquées pour tous les détecteurs de rayonnement ou avec une zone des détecteurs de rayonnement, dans lequel la zone des détecteurs de rayonnement est sélectionnée pour une distance prédéterminée dans chaque cas de sorte que les amplitudes des signaux sont similaires pour toutes les distances prédéterminées,

dans lequel l'une des longueurs d'onde irradiées ou une plage de longueurs d'onde est appropriée pour avoir un effet sur les signaux de détection de la substance cible à déterminer, c'est-à-dire les quantités de rayonnement détectées par le(s) détecteur(s), et

dans lequel pour la détection de substances perturbatrices ou d'autres influences perturbatrices au moins une des longueurs d'onde irradiées ou une des plages de longueurs d'onde irradiées est appropriée pour n'avoir aucun effet sur les signaux de détection de la substance cible à déterminer, c'est-à-dire les quantités de rayonnement détectées par le(s) détecteur(s),

dans lequel la substance cible est une ou plusieurs substances issues du groupe des antioxydants, de l'eau, de l'hémoglobine, de l'hémoglobine oxydée, de l'hématocrite, des graisses, des protéines, de la mélanine, de la crème solaire et de la bilirubine,

dans lequel toujours au moins une paire de longueurs d'onde du rayonnement, montrant un effet et ne montrant aucun effet, peut être irradiée à la même distance d.

2. Dispositif de détection selon la revendication 1, dans lequel la barrière anti-rayonnement comprend un substrat portant des composants et/ou un boîtier entourant la source de rayonnement (LED1-LED6) et bloque ainsi la transmission du rayonnement dans le substrat et/ou la transmission du rayonnement à travers le boîtier.

3. Dispositif de détection selon la revendication 1 ou 2, dans lequel au moins une source de rayonnement (LED1-LED6) est conçue et/ou agencée de sorte que le rayonnement de la source de rayonnement traverse l'objet de mesure jusqu'à une profondeur prédéterminée.

4. Dispositif de détection selon au moins l'une des revendications 1 à 3, dans lequel les distances entre au moins deux des détecteurs de rayonnement (PD1-PD4) et les sources de rayonnement (LED1-LED6) sont choisies de sorte que la longueur de trajet du rayonnement dans le volume cible pour l'au moins une substance cible à détecter est maximisée et les volumes de mesure pour les différents détecteurs de rayonnement se chevauchent au moins partiellement.

5. Dispositif de détection selon au moins l'une des revendications 1 à 4, dans lequel les sources de rayonnement (LED1-LED6) et les détecteurs de rayonnement (PD1-PD4) sont disposés de sorte qu'une pondération en profondeur est sélectionnée en fonction des différentes distances de sorte que l'influence des volumes perturbateurs proches de la surface ou l'influence des volumes inférieurs au volume cible est réduite en décalant les signaux à différentes distances.

6. Dispositif de détection selon au moins l'une des revendications 1 à 5, dans lequel au moins une des sources de rayonnement (LED1-LED6) est conçue de sorte qu'au moins une longueur d'onde ou une plage de longueurs d'ondes peut être irradiée pour chaque grandeur perturbatrice et qu'en utilisant plus d'une longueur d'onde ou d'une plage de longueurs d'ondes dans le dispositif de détection, la sensibilité pour cette substance est augmentée et/ou que des sous-classes de la substance cible sont résolues.

7. Dispositif de détection selon au moins l'une des revendications 1 à 6, dans lequel le dispositif de détection présente une unité de réglage de la température qui est conçue pour chauffer et/ou refroidir les sources de rayonnement (LED1-LED6) de sorte que la température des sources de rayonnement peut être réglée à une valeur de température prédéterminée.

8. Dispositif selon les revendications 1 à 7, dans lequel le dispositif de détection est conçu pour effectuer une série de mesures sur des objets de mesure ou sur des mélanges de substances ayant des propriétés analogues à celles de l'objet de mesure en ce qui concerne la substance cible et les grandeurs perturbatrices, dans laquelle série de mesure la concentration de la substance cible peut être modifiée de manière ciblée comme valeur de référence ou est connue au préalable par un autre procédé pour différents objets de mesure et pour lesquels les perturbations peuvent être modifiées et une règle d'étalonnage peut être déterminée, laquelle permet de prédire la concentration de la substance cible et donc le dispositif de détection est disponible en tant que dispositif de détection de référence avec un étalonnage de référence pour un transfert d'étalonnage ultérieur.

9. Dispositif de détection selon la revendication 8, dans lequel le dispositif de détection présente un moyen de transmission d'étalonnage et/ou peut être connecté à un moyen de transmission d'étalonnage, qui est conçu pour transmettre l'étalonnage de référence à d'autres dispositifs de détection structurellement similaires en effectuant des mesures de comparaison avec le dispositif de détection de référence et les dispositifs de détection structurellement similaires, et une fonction de transmission de signal du dispositif de détection structurellement similaire peut être ajustée si l'écart est trop grand.

10. Dispositif de détection selon au moins l'une des revendications 1 à 9, dans lequel le dispositif de détection est conçu pour, pendant le fonctionnement, mesurer des substances cibles dans la peau d'un utilisateur et dans lequel les substances cibles à mesurer dans la peau sont des antioxydants, c'est-à-dire des flavonoïdes ou des caroténoïdes, en particulier le bêta-carotène, le lycopène, la lutéine, la zéaxanthine ou la capsanthine, qui doivent être mesurés dans l'épiderme et le derme, ou les substances cibles à mesurer sont les graisses, l'eau et les protéines présentes dans les tissus animaux ou les produits carnés, qui sont présents soit tels que cultivés soit après un processus de traitement, ou la substance cible à mesurer est la mélanine, qui doit être mesurée dans la peau au niveau de l'épiderme, ou la substance cible à mesurer est la bilirubine, qui doit être mesurée dans la peau au niveau du derme, ou la substance cible à mesurer dans la peau est l'eau, qui est présente dans la peau à différentes concentrations de l'épiderme, du derme et du sous-cutané et doit être évaluée en tant que rétention d'eau en fonction du temps dans l'insuffisance cardiaque, pour une évaluation d'un apport hydrique adéquat ou pour une évaluation de la fonction rénale.

11. Dispositif de détection selon au moins l'une des revendications 1 à 9, dans lequel le dispositif de détection est conçu pour, pendant le fonctionnement, mesurer les substances cibles que sont l'hémoglobine et/ou l'hémoglobine oxygénée et/ou pour déterminer l'hématocrite dans le sang dans une circulation sanguine extracorporelle pendant la dialyse ou l'aphérèse du sang ou autre situation dans laquelle une partie du sang se trouve dans un système de tubulures ou une cuvette comme point de mesure.

12. Dispositif de détection selon la revendication 10 ou 11, dans lequel les sources de rayonnement (LED1-LED6) et les détecteurs de rayonnement (PD1-PD4) sont chacun isolés de l'objet de mesure par une fenêtre et il existe entre eux une barrière anti-rayonnement qui affleure les fenêtres vers l'objet de mesure ou qui peut dépasser de celles-ci.

**13.** Dispositif de détection selon l'une des revendications 10 à 12, dans lequel le dispositif de détection est conçu pour évaluer des substances cibles séparément pour différentes sous-classes en sélectionnant une source de rayonnement (LED1-LED6) pour chaque sous-classe de sorte que sa longueur d'onde centrale correspond à une longueur d'onde d'un maximum d'absorption de la sous-classe de substances cibles.

**14.** Dispositif de détection selon au moins l'une des revendications 10 à 13, dans lequel le dispositif de détection est conçu pour déterminer une valeur prédite de la substance cible à partir de la mesure de la substance cible et dans lequel le dispositif de détection présente une interface de communication qui est connectée ou peut être connectée à un appareil de sortie et l'interface de communication est formée pour transmettre la valeur prédite de la substance cible à l'appareil de sortie.

**15.** Dispositif de détection selon au moins l'une des revendications 10 à 14, dans lequel le dispositif de détection est conçu pour évaluer séparément une teneur en eau sur la base de différences dépendant de la longueur d'onde dans une profondeur de pénétration pour le derme et le sous-cutané, en prévoyant des sources de rayonnement (LED1-LED6) avec une faible absorption d'eau et donc une profondeur de pénétration élevée pour le sous-cutané et en prévoyant des sources de rayonnement avec une absorption d'eau plus élevée et donc une profondeur de pénétration plus faible pour le derme.

**16.** Procédé de détection à résolution spatiale de substances cibles comprenant les étapes suivantes :

irradiation de lumière à partir de plusieurs sources de rayonnement (LED1-LED6) ou à partir d'une source ayant plusieurs plages de longueurs d'onde et

détection de valeurs de mesure dans plusieurs détecteurs de rayonnement (PD1-PD4) ou dans un détecteur de rayonnement ayant plusieurs sous-unités lisibles séparément pour déterminer les quantités de substances cibles dans des objets de mesure hautement diffusants,

disposition d'une barrière anti-rayonnement,

dans lequel la barrière anti-rayonnement est conçue pour absorber et/ou réfléchir un rayonnement d'au moins une plage de longueurs d'onde, dans lequel la disposition des sources de rayonnement est effectuée à différentes distances prédéterminées des détecteurs de rayonnement et dans lequel les sources de rayonnement sont disposées séparées des détecteurs de rayonnement par la barrière anti-rayonnement de sorte que les rayonnements générés par les sources de rayonnement, avant de tomber sur les détecteurs de rayonnement, parcourent d'abord une longueur de trajet à travers l'objet de mesure,

amplification des signaux des détecteurs de rayonnement de sorte que des amplitudes de signal similaires sont obtenues pour tous les détecteurs de rayonnement, ou

sélection de la surface des détecteurs de rayonnement de sorte que des amplitudes de signal similaires sont obtenues pour tous les détecteurs de rayonnement,

dans lequel l'une des longueurs d'onde ou une plage de longueurs d'onde qui a un effet sur les signaux de détection de la substance cible à déterminer, c'est-à-dire les quantités de rayonnement détectées par le(s) détecteur(s), et

dans lequel l'une des longueurs d'onde ou une plage de longueurs d'onde pour la détection de substances perturbatrices ou d'autres influences perturbatrices qui n'ont pas d'effet sur les signaux de détection de la substance cible à déterminer, c'est-à-dire les quantités de rayonnement détectées par le(s) détecteur(s),

dans lequel la substance cible est une ou plusieurs substances issues du groupe des antioxydants, de l'eau, de l'hémoglobine, de l'hémoglobine oxydée, de l'hématocrite, des graisses, des protéines, de la mélanine, de la crème solaire et de la bilirubine,

dans lequel le rayonnement de toujours au moins une paire de longueurs d'onde du rayonnement, montrant un effet et ne montrant aucun effet, peut être effectuée à la même distance d.

**Fig. 1**

PD 4

PD 3

PD 2     16      15      14      13      12      11      10      9

PD 1

LED 1

LED 2

LED 3

Monitor PD

Pin 1     2       3       4       5       6       7       8

LED 6

LED 5

LED 4

**Fig. 2**

PD 4

PD 3

PD 2

PD 1

LED 1

LED 2

Temperatursensor

**Fig. 3**

Lösung A

Lösung B

Lösung C

Lösung D

Lösung E

Lösung F

Lösung G

Lösung H

Lösung I

Lösung J

Lösung K

Lösung L

**Fig. 4**

**Fig. 5**

PD 1

PD 2

PD 3

PD 4

Monitor PD

LED 3

LED 6

0,90

0,74

1,61

1,62

0,74

A

0,37

A

1,33

LED 4

LED 5

LED 1

LED 2

Temperatursensor

0,40

0,64

0,80

0,20

0,75

A-A

**Fig. 6**

**Fig. 7**

**Fig. 8**

**Fig. 9**

**Fig. 10**

Lichtwellenleiter
Metallrohr
Messkopf
Haut

**Fig. 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013049677 A1
- EP 0760091 B1
- EP 0756848 B1
- DE 102007054309

**In der Beschreibung aufgeführte Nicht-Patentliteratur**

- **R.A. BOLT ; K.R. RINZEMA ; J.J. TEN BOSCH.** *Pure Appl. Opt.,* 1995, vol. 4
- **S. FENG ; F.-A. ZENG ; B. CHANCE.** *Appl. Opt.,* 1995, vol. 34 (19